# EUROPEAN PATENT APPLICATION

(11) **EP 1 195 371 A1**
(43) Date of publication of application: **10.04.2002**
(21) Application number: 00937319.2
(22) Date of filing: 20.06.2000
(51) Int. Cl.: C07D 209/88, C07D 303/36, C07D 307/91, C07D 333/76, C07D 301/24, C07C 303/40, C07C 311/08, C07C 311/13, A61K 31/403, A61P 3/06, A61P 3/10

(54) **PROCESS FOR THE PREPARATION OF TRICYCLIC AMINO ALCOHOL DERIVATIVES**

(30) Priority: 09.07.1999 JP 19551999
(71) Applicant: Asahi Kasei Kabushiki Kaisha, Osaka-shi, Osaka 530-8205 (JP)
(72) Inventor: MIYOSHI, Shiro, Fuji-shi, Shizuoka 416-0946 (JP); MATSUBARA, Koki, Fuji-shi, Shizuoka 416-0933 (JP)
(74) Representative: Forstmeyer, Dietmar, Dr. rer. nat., Dipl.-Chem.
(86) International application number: JP0004015
(87) International publication number: WO0104092

(57) **Abstract**

A process for the preparation of tricyclic amino alcohol derivatives of formula (1) or salts thereof which are useful in the treatment and prevention of diabetes, obesity, hyperlipidemia and so on; and intermediates useful for the process: 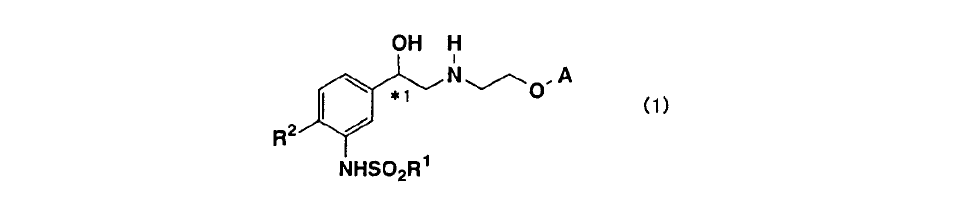 wherein R¹ is lower alkyl group or benzyl; *1 represents an asymmetric carbon atom; R² is hydrogen, halogen, or hydroxyl; and A is one of the following groups: 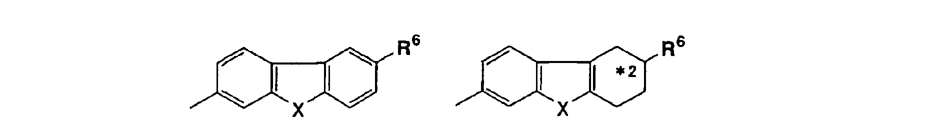 (wherein X is NH, O or S; R⁶ is hydrogen, hydroxyl, amino, or acetylamino; and *2 represents an asymmetric carbon atom when R⁶ is not hydrogen)

## Description

### FIELD OF THE INVENTION

The present invention relates to a novel process for the preparation of tricyclic amino alcohol derivatives having the formula (1): 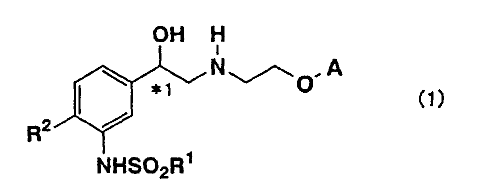 wherein
R¹ represents a lower alkyl group or a benzyl group;
*1 represents an asymmetric carbon atom;
R² represents a hydrogen atom, a halogen atom or a hydroxyl group; and
A represents one of the following groups: 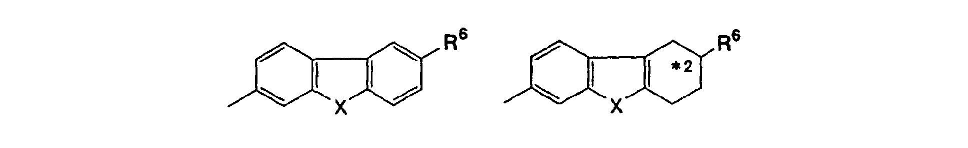 wherein X represents NH, O or S; R⁶ represents a hydrogen atom, a hydroxyl group, an amino group or an acetylamino group; and *2 represents an asymmetric carbon atom when R⁶ is not a hydrogen atom,
or salts thereof, which are useful for treating and preventing diabetes, obesity, hyperlipidemia and the like; as well as to intermediates useful for the process.

### BACKGROUND OF THE INVENTION

JP-A-9-249623 (WO97/25311) and WO99/01431 disclose in detail processes for the preparation of compounds of the abovementioned formula (1) and also describe that these compounds are very useful for treating and preventing diabetes, obesity, hyperlipidemia and the like.

However, the study on the above known processes carried out by the present inventors has shown that these processes are not necessarily practical. There would be a need for a more convenient, practical preparation process with low cost which comprises a small number of steps with good industrial work efficiency.

### DISCLOSURE OF THE INVENTION

The study carried out by the present inventors showed some disadvantages involved in the conventional processes for preparing a compound of the formula (1) set forth above, wherein the disadvantages were that the processes require many reaction steps and several purifying works including chromatography, and did not necessarily provide a good yield. In addition, if an optical isomer, such as R-form, of a compound of the formula (1) is to be finally obtained according to the synthesizing route disclosed in the above patent publications, the carbonyl group should be reduced with a borane as a reducing agent in the presence of a chiral auxiliary agent of the following formulae:

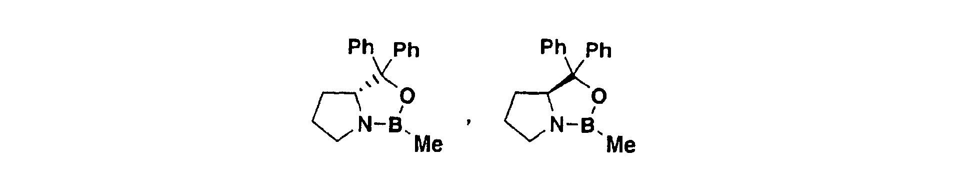

This chiral auxiliary agent is very expensive and the process for the preparation thereof is very complicated. The chiral auxiliary agent is a hazardous, combustible substance and an asymmetric reduction using the said chiral auxiliary agent requires strictly anhydrous conditions, strict temperature controls, complicated works and the like, which will become problematic when the chiral auxiliary agent is industrially used.

In order to solve the above problems, the present inventors have examined a variety of synthesizing processes. As a result, the present inventors have established preferred synthesizing processes and completed the present invention.

That is, the present invention is a process for the preparation of a compound of the formula (1): 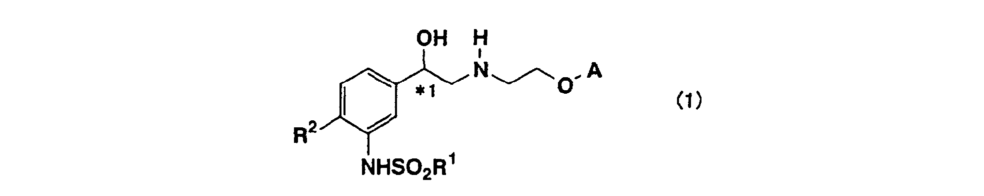 wherein R¹ represents a lower alkyl group or a benzyl group; R² represents a hydrogen atom, a halogen atom or a hydroxyl group; *1 represents an asymmetric carbon atom; and A represents one of the following groups: 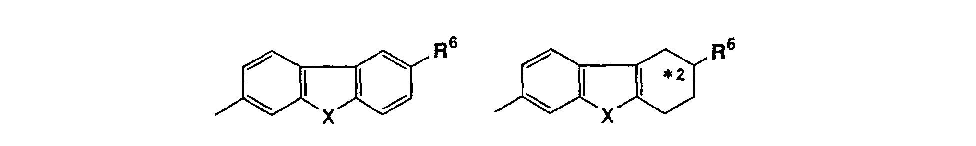 wherein X represents NH, O or S; R⁶ represents a hydrogen atom, a hydroxyl group, an amino group or an acetylamino group; and *2 represents an asymmetric carbon atom when R⁶ is not a hydrogen atom,
said process comprising:
chlorinating a compound of the formula (7): 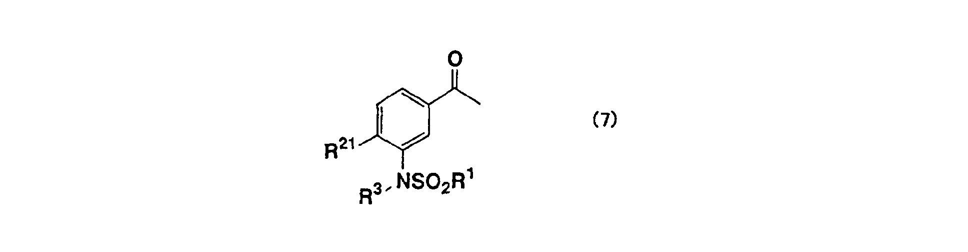 wherein R¹ is as defined above; R²¹ represents a hydrogen atom, a halogen atom or a protected hydroxyl group; and R³ represents an amino-protecting group, to give a compound of the formula (6): 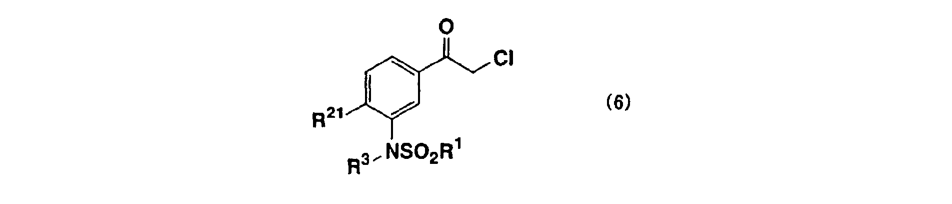 wherein R¹, R²¹ and R³ are as defined above; and then,
reducing the compound of the formula (6) to give a chlorohydrin compound of the formula (5): 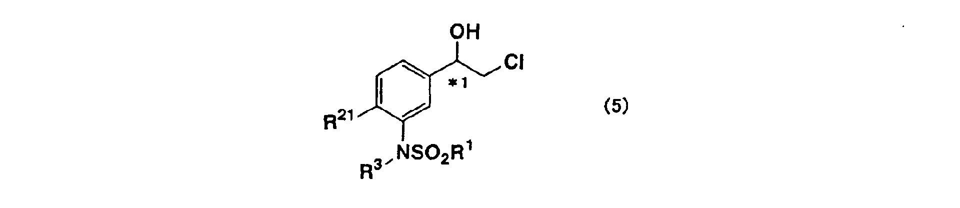 wherein R¹, R²¹, R³ and *1 are as defined above;
and then,
(i) treating the chlorohydrin compound with an alkali to give an epoxy compound of the formula (4): 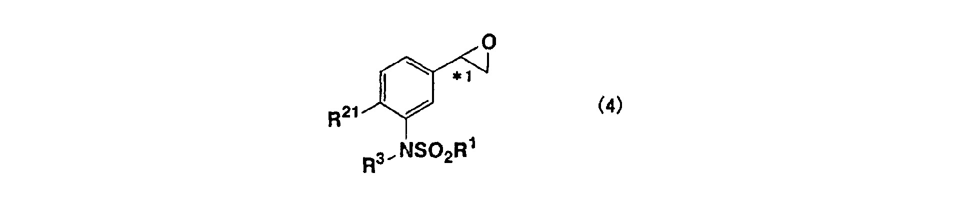 wherein R¹, R²¹, R³ and *1 are as defined above; and then,
   (i-a) reacting the epoxy compound of the formula (4) with a compound of the formula (8): 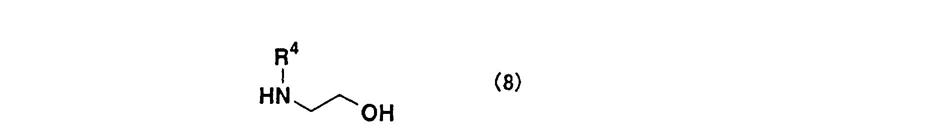 wherein R⁴ represents an amino-protecting group, to give a dialcohol compound of the formula (3): 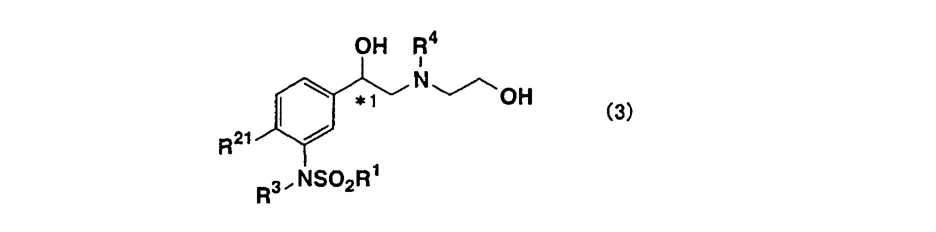 wherein R¹, R²¹, R³, R⁴ and *1 are as defined above;
      brominating the primary alcohol, and then reacting the brominated product with a compound represented by A'-OH wherein A' represents one of the following groups: 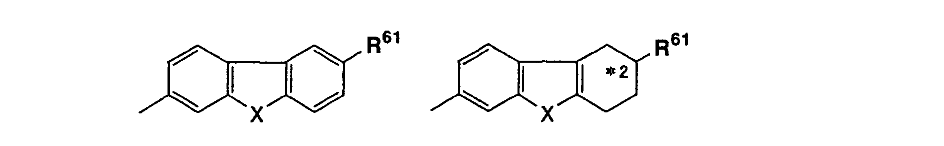 wherein X represents NH, O or S; R⁶¹ represents a hydrogen atom, a protected hydroxyl group, a protected amino group or an acetylamino group; and *2 represents an asymmetric carbon atom when R⁶¹ is not a hydrogen atom, to give an amino alcohol compound of the formula (2): 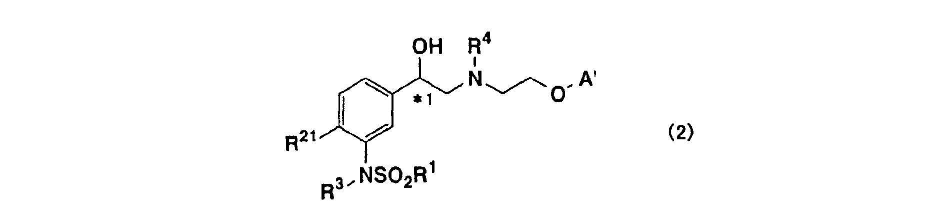 wherein R¹, R²¹, R³, R⁴, A' and *1 are as defined above; or
   (i-b) reacting the compound of the formula (4) with a compound of the formula (9): 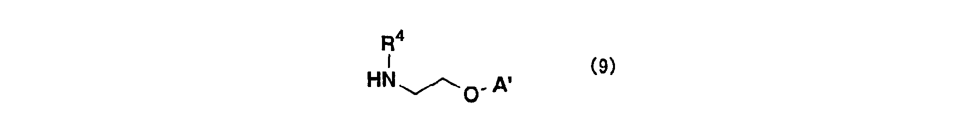 wherein R⁴ and A' are as defined above, to give a compound of the formula (2);
   or, alternatively,
(ii) protecting the hydroxyl group of the compound of the formula (5) with a protecting group R⁵, or halogenating the compound of the formula (5) followed by protecting the hydroxyl group with a protecting group R⁵ to give a compound of the formula (10): 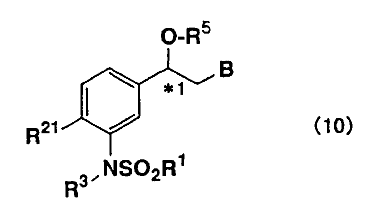 wherein R⁵ represents a hydroxyl-protecting group; B represents a halogen atom; and R¹, R²¹, R³ and *1 are as defined above; and then,
   (ii-a) reacting the compound of the formula (10) with the compound of the formula (8) to give an alcohol compound of the formula (3a): 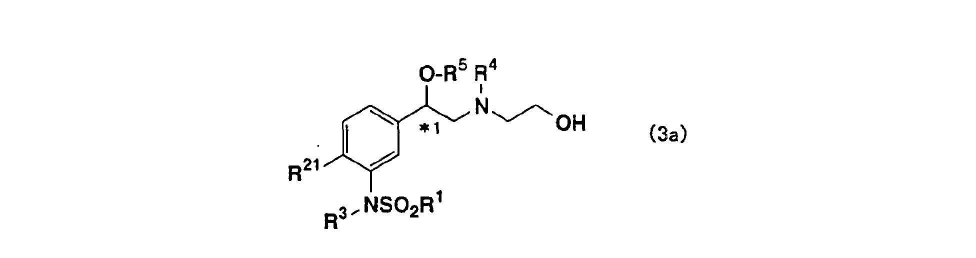 wherein R¹, R²¹, R³, R⁴, R⁵ and *1 are as defined above;
      brominating the alcohol, and then reacting the brominated product with a compound represented by the A'-OH set forth above to give an amino alcohol compound of the formula (2a): 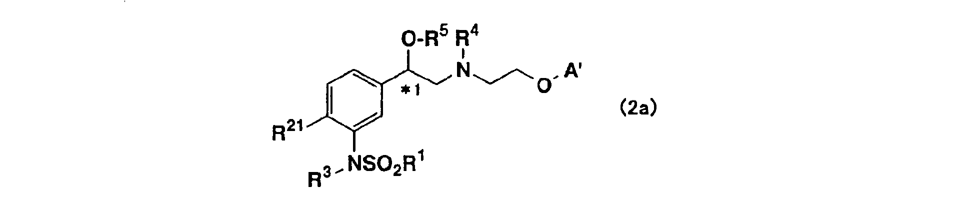 wherein R¹, R²¹, R³, R⁴, R⁵, A' and *1 are as defined above; or
   (ii-b) reacting the compound of the formula (10) with the compound of the formula (9) to give an amino alcohol compound of the formula (2a);
and then,
simultaneously or sequentially removing the protecting groups of the thus obtained compound of the formula (2) or (2a) to give a compound of the formula (1).

The first synthesizing route of the present invention is a process for the preparation of a compound of the formula (1): 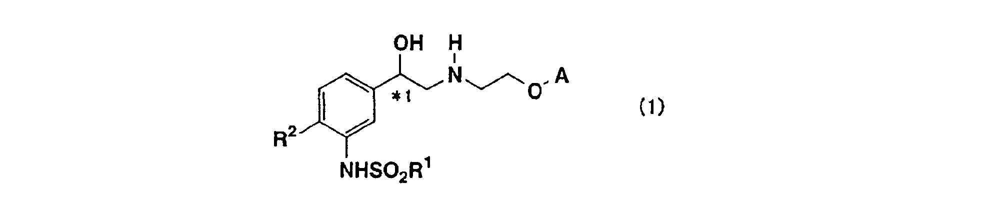 wherein R¹, R², *1 and A are as defined above,
said process comprising:
chlorinating a compound of the formula (7): 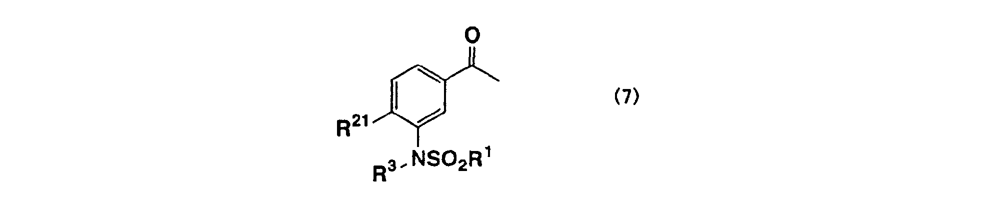 wherein R¹, R²¹ and R³ are as defined above, to give a compound of the formula (6): 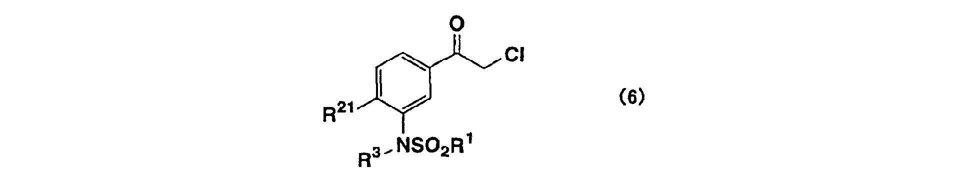 wherein R¹, R²¹ and R³ are as defined above; and then,
reducing the compound of the formula (6) to give a chlorohydrin compound of the formula (5): 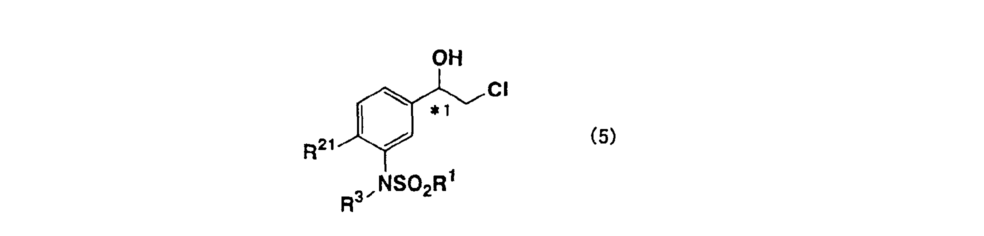 wherein R¹, R²¹, R³ and *1 are as defined above; and then,
treating the chlorohydrin compound with an alkali to give an epoxy compound of the formula (4): 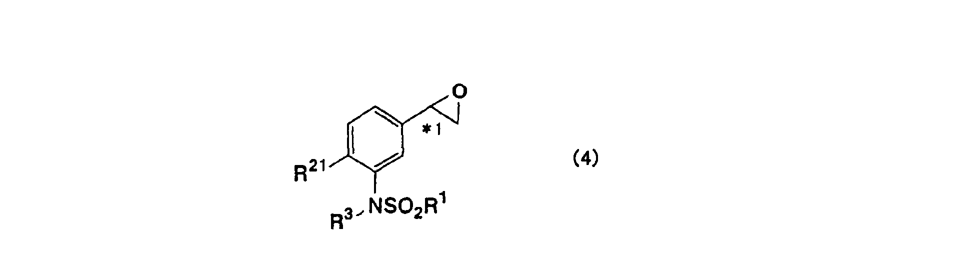 wherein R¹, R²¹, R³ and *1 are as defined above; and then,
reacting the epoxy compound of the formula (4) with a compound of the formula (8): 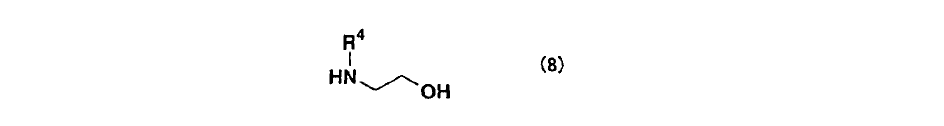 wherein R⁴ is as defined above; to give a dialcohol compound of the formula (3): 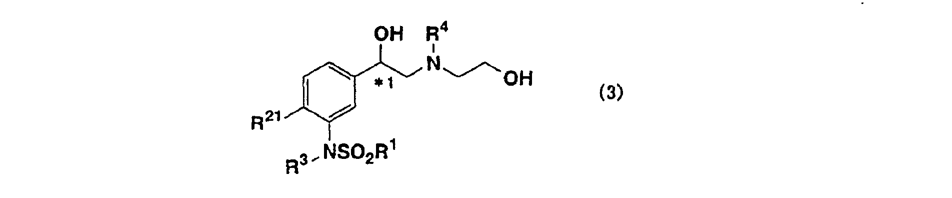 wherein R¹, R²¹, R³, R⁴ and *1 are as defined above;
brominating the primary alcohol, and then reacting the brominated product with a compound represented by A'-OH wherein A' is as defined above; to give an amino alcohol compound of the formula (2): 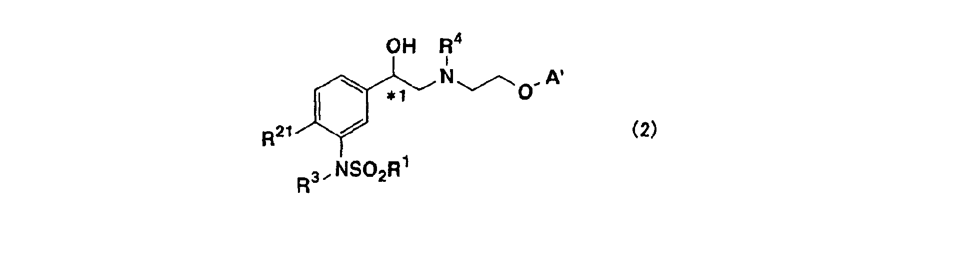 wherein R¹, R²¹, R³, R⁴, A' and *1 are as defined above; and then, simultaneously or sequentially removing the protecting groups of the compound of the formula (2) to give a compound of the formula (1).

In the aspect of the first synthesizing route set forth above, compounds of the formulae (6), (5) and (4) are preferred intermediates which are novel and relatively good in crystallinity. These compounds do not necessarily need a column chromatography purifying step and may be used in the following reaction step after being subjected to a recrystallizing treatment and the like. In addition, compounds of the formulae (5) and (4), each of which can be improved in its optical purity by recrystallizing treatment, are useful intermediates. Specific examples of a compound of the formula (6) include 2-chloro-1-[3-(N-benzyl-N-methylsulfonylamino)phenyl]ethanone; 2-chloro-1-[4-benzyloxy-3-(N-benzyl-N-methylsulfonylamino)phenyl]ethanone; 2-chloro-1-[4-chloro-3-(N-benzyl-N-methylsulfonylamino)phenyl]ethanone; 2-chloro-1-[4-bromo-3-(N-benzyl-N-methylsulfonylamino)phenyl]ethanone and the like. Specific examples of a compound of the formula (5) include (R)-2-chloro-1-[3-(N-benzyl-N-methylsulfonylamino)phenyl]ethanol; (R)-2-chloro-1-[4-benzyloxy-3-(N-benzyl-N-methylsulfonylamino)phenyl]ethanol; (R)-2-chloro-1-[4-chloro-3-(N-benzyl-N-methylsulfonylamino)phenyl]ethanol; and (R)-2-chloro-1-[4-bromo-3-(N-benzyl-N-methylsulfonylamino)phenyl]ethanol, racemate thereof and the like.

Specific examples of a compound of the formula (4) include (R)-1-[3-(N-benzyl-N-methylsulfonylamino)phenyl]oxirane; (R)-1-[4-benzyloxy-3-(N-benzyl-N-methylsulfonylamino)phenyl]oxirane; (R)-1-[4-chloro-3-(N-benzyl-N-methylsulfonylamino)phenyl]oxirane; and (R)-1-[4-bromo-3-(N-benzyl-N-methylsulfonylamino)phenyl]oxirane, racemate thereof and the like.

In the steps set forth above, the step in which a compound of the formula (6) is reduced to give a compound of the formula (5) is specifically characteristic of the present process.

In addition, when one of optical isomers of a compound of the formula (1) is to be obtained in the steps set forth above, a compound of the formula (6) is preferably subjected to an asymmetrical reduction. In this case, the resulting chlorohydrin compound of the formula (5), and the resulting compounds of the formulae (4), (3), (2) and (1) are obtained as one of their optical isomers, respectively. This step is also characteristic of these steps.

In the first synthesizing route set forth above, a compound of the formula (3) is also a preferred intermediate which is novel. This compound does not necessarily need a column chromatography purifying step and may be used in the following reaction step after being subjected to a recrystallizing treatment and the like.

Specific examples of a compound of the formula (3) include (R)-2-[N'-benzyl-N'-(2-hydroxyethyl)amino]-1-[3-(N-benzyl-N-methylsulfonylamino)phenyl]ethanol; (R)-2-[N'-benzyl-N'-(2-hydroxyethyl)amino]-1-[4-benzyloxy-3-(N-benzyl-N-methylsulfonylamino)phenyl]ethanol; (R)-2-[N'-benzyl-N'-(2-hydroxyethyl)amino]-1-[4-chloro-3-(N-benzyl-N-methylsulfonylamino)phenyl]ethanol; (R)-2-[N'-benzyl-N'-(2-hydroxyethyl)amino]-1-[4-bromo-3-(N-benzyl-N-methylsulfonylamino)phenyl]ethanol, racemate thereof and the like.

In the first synthesizing route set forth above, the coupling reaction of a compound of the formula (4) and a compound of the formula (8) is preferred when R²¹ represents a hydrogen or halogen atom.

The second synthesizing route of the present invention for the preparation of a compound of the formula (1) comprises reacting a compound of the formula (4) set forth above with a compound of the formula (9): 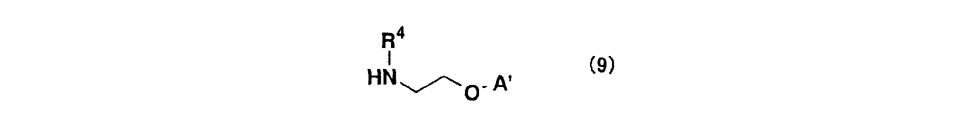 wherein R⁴ and A' are as defined above; and treating the thus obtained compound of the formula (2) as set forth above to give a compound of the formula (1).

In the step set forth above, the step in which a compound of the formula (4) is reacted with a compound of the formula (9) to give a compound of the formula (2) is characteristic of the present process. Further, these steps are characterized by involving a smaller numbers of steps as compared with the first synthesizing route.

The second synthesizing route set forth above is preferred when R²¹ represents a hydrogen or halogen atom.

The third synthesizing route of the present invention is a process for the preparation of a compound of the formula (1), said process comprising:
protecting the hydroxyl group of the compound of the formula (5) with a protecting group R⁵, or halogenating the compound of the formula (5) followed by protecting the hydroxyl group with a protecting group R⁵ to give a compound of the formula (10): 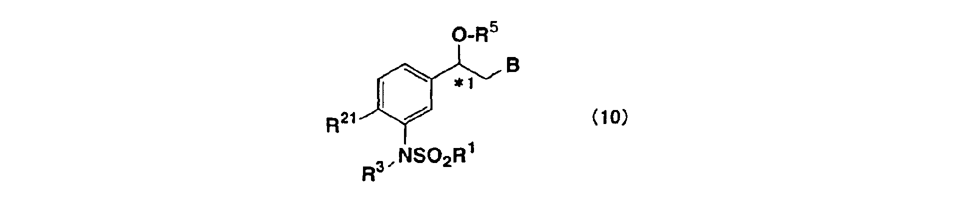 wherein R¹, R²¹, R³, R⁵, B and *1 are as defined above; and,
reacting the compound of the formula (10) with a compound of the formula (8) set forth above to give an alcohol compound of the formula (3a): 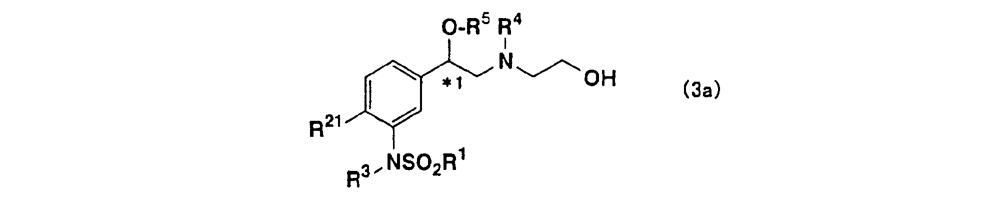 wherein R¹, R²¹, R³, R⁴, R⁵ and *1 are as defined above;
brominating the primary alcohol, and then reacting the brominated product with a compound represented by A'-OH set forth above to give an amino alcohol compound of the formula (2a): 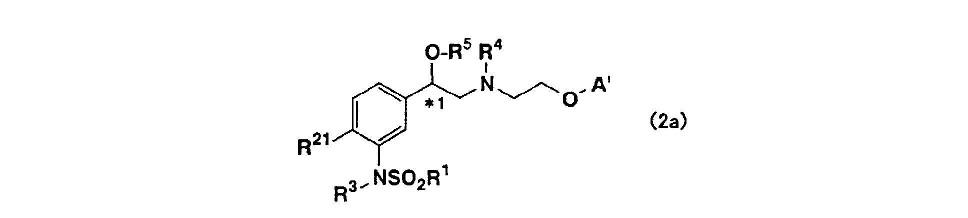 wherein R¹, R²¹, R³, R⁴, R⁵, A' and *1 are as defined above; and treating the thus obtained compound in the same way as aforesaid to give a compound of the formula (1).

According to this synthesizing route, a compound of the formula (10) is novel and relatively good in crystallinity. This compound can be purified by recrystallization and is useful intermediate to improved optical purity of a compound of the present invention.

Specific examples of a compound of the formula (10) include (R)-N-benzyl-N-[3-[2-iodo-1-[(triethylsilyl)oxy]ethyl]phenyl]methanesulfonamide; (R)-N-benzyl-N-[5-[2-iodo-1-[(triethylsilyl)oxy]ethyl]-2-(benzyloxy)phenyl]methanesulfonamide; (R)-N-benzyl-N-[5-[2-iodo-1-[(triethylsilyl)oxy]ethyl]-2-chlorophenyl]methanesulfonamide; (R)-N-benzyl-N-[5-[2-iodo-1-[(triethylsilyl)oxy]ethyl]-2-bromophenyl]methanesulfonamide, racemate thereof and the like.

In the step set forth above, the step in which a compound of the formula (10) is reacted with a compound of the formula (8) to give a compound of the formula (3a) is characteristic of the present process.

The fourth synthesizing route of the present invention is a process for the preparation of a compound of the formula (1), which process comprises reacting a compound of the formula (10) set forth above with a compound of the formula (9) set forth above to give an amino alcohol compound of the formula (2a); and treating the thus obtained compound as set forth above to give a compound of the formula (1).

This synthesizing route is characterized by the step of reacting a compound of the formula (10) with a compound of the formula (9). Further, this synthesizing route is characterized by involving a smaller number of steps as compared with the third synthesizing route.

This specification includes all of the contents as disclosed in the specification and/or drawings of Japanese Patent Application No. 11-195519, which is the base of the priority right of the present application.

### PREFERRED EMBODIMENT OF THE INVENTION

As used herein, R²¹ and R² may be a hydrogen atom, a halogen atom or a hydroxyl group (a protected hydroxyl group in case of R²¹), with hydrogen being particularly preferred. A halogen atom is also preferred as R²¹ and R². The halogen atom may be fluorine, chlorine, bromine or iodine, with chlorine and bromine being particularly preferred.

B is a halogen atom. Generally, B is preferably chlorine, bromine, or iodine.

The term "lower alkyl" means a straight or branched saturated hydrocarbon containing 1 to 6 carbon atoms and may be preferably a straight or branched alkyl group, such as methyl, ethyl, n-propyl, i-propyl, n-butyl, i-butyl, sec-butyl, tert-butyl, pentyl, hexyl and the like, or a cycloalkyl group such as cyclopropyl, cyclobutyl, cyclopentyl, cyclohexyl and the like. Methyl is particularly preferred.

R¹ may be preferably the alkyl group set forth above, with methyl being particularly preferred. A benzyl group may be also preferred as R¹.

R³ and R⁴ are an amino-protecting group. Examples of the amino-protecting group include an acyl group, an acyloxy group, an easily removable aralkyl group and the like. The easily removable aralkyl group may be benzyl, substituted benzyl, naphthylmethyl, substituted naphthylmethyl and the like, with benzyl being particularly preferred. The aralkyl group to be used may be an aralkyl group containing 7 to 16 carbon atoms. Specific examples thereof include benzyl, phenethyl, 3-phenylpropyl, 4-phenylbutyl, (1-naphthyl)methyl, 2-(1-naphthyl)ethyl, 2-(2-naphthyl)ethyl and the like, wherein the phenyl or naphthyl moiety may have suitable substituents such as alkyl, alkoxy, halogen and the like on suitable positions.

A carbazole group is particularly preferred as A.

R⁶ may be preferably a hydrogen atom. A hydroxyl group is also preferred as R⁶. R⁶¹ may be preferably a hydrogen atom. A protected hydroxyl group is also preferred as R⁶¹.

Each compound of the formulae (1), (2), (2a), (3), (3a), (4), (5) and (10), in which formulae * 1 means an asymmetric carbon atom, can be in the form of two optical isomers. Therefore, not only optically pure isomers of these compounds, but also a mixture of any two isomers are encompassed in the present invention. From the viewpoint of pharmacological activity, a preferred configuration of the asymmetric carbon may be the absolute configuration R.

*2 means an asymmetric carbon atom and a compound containing *2 can be in the form of two optical isomers. Therefore, not only optically pure isomers of these compounds, but also a mixture of any two isomers are encompassed in the present invention.

R²¹ and R⁵ are a hydroxyl-protecting group. The hydroxyl-protecting group is not limited as long as it is commonly used as a hydroxyl-protecting group. Preferred examples of easily and selectively removable protecting group generally include, for example, a trialkylsilyl group, an alkoxyalkyl group, an acyl group and the like. These hydroxyl-protecting groups can be introduced and removed by a known method indicated in literatures accepted in the art (for example, T. W. Greene, P. G. M. Wuts, et al., Protective Groups in Organic Synthesis, Wiley-Interscience Publication). For example, a tert-butyldimethylsilyl group (TBDMS) may be introduced into the alcohol by the treatment of the alcohol with a silylating agent such as tert-butyldimethylchlorosilane, tert-butyldimethylsilyl trifluoromethanesulfonate and the like in the presence of an acid scavenger. The amount of the silylating agent to be added may be generally about 1.0 to 1.5 mol for 1 mol of the alcohol. Generally, this reaction is preferably carried out in an inert medium. The inert medium may be dichloromethane, tetrahydrofuran, acetonitrile, pyridine and the like. The inert medium may be preferably N,N-dimethylformamide. The amount of the inert medium to be used may be about 1 to 5 mL for 1 g of the alcohol. The acid scavenger may be triethylamine, N,N-diisopropylethylamine, pyridine, N,N-dimethylaminopyridine and the like. The acid scavenger may be preferably imidazole. The amount of the acid scavenger to be added may be generally about 1 to 3 mol for 1 mol of the alcohol. Generally, this reaction is preferably carried out at a temperature of about -20°C to 80°C, particularly about 0°C to room temperature, for example, for 1 to 5 hours.

A benzyloxymethyl group (BOM) may be introduced into the alcohol by the treatment of the alcohol with chloromethyl benzyl ether in the presence of an acid scavenger. The amount of chloromethyl benzyl ether to be added may be generally about 1.0 to 1.5 mol for 1 mol of the alcohol. Generally, this reaction is preferably carried out in an inert medium. The inert medium may be tetrahydrofuran, acetonitrile, N,N-dimethylformamide and the like. The inert medium may be preferably dichloromethane. The amount of the inert medium to be used may be about 1 to 5 mL for I g of the alcohol. The acid scavenger may be triethylamine, pyridine, N,N-dimethylaminopyridine and the like. The acid scavenger may be preferably N,N-diisopropylethylamine. The amount of the acid scavenger to be added may be generally about 1 to 3 mol for 1 mol of the alcohol. Generally, this reaction is preferably carried out at a temperature of about -20°C to 80°C, particularly about 0°C to room temperature, for example, for 1 to 5 hours.

In addition, an acetyl group (Ac) may be introduced into the alcohol by the treatment of the alcohol with an acetylating agent such as acetic anhydride, acetyl chloride and the like in the presence of an acid scavenger. The amount of the acetylating agent to be added may be generally about 1 to 3 mol for 1 mol of the alcohol. Generally, this reaction is preferably carried out in an inert medium. The inert medium may be preferably tetrahydrofuran, acetonitrile, dichloromethane, pyridine and the like. The amount of the inert medium to be used may be about 1 to 5 mL for 1 g of the alcohol. The acid scavenger may be preferably triethylamine, N,N-diisopropylethylamine, pyridine, N,N-dimethylaminopyridine and the like. The amount of the acid scavenger to be added may be generally about 1 to 3 mol for 1 mol of the alcohol. Generally, this reaction is preferably carried out at a temperature of about -20°C to 80°C, particularly about 0°C to room temperature, for example, for 1 to 5 hours.

The hydroxyl-protecting group may be removed as follows. For example, the tert-butyldimethylsilyl group may be removed by the treatment of the tert-butyldimethylsilyl ether with tetrabutylammonium fluoride. The amount of tetrabutylammonium fluoride to be added may be generally about 1 to 3 mol for 1 mol of the tert-butyldimethylsilyl ether. Generally, this reaction is preferably carried out in a medium such as tetrahydrofuran and the like. The amount of the medium to be used may be generally about 1 to 5 mL for 1 g of the alcohol. Generally, this reaction is preferably carried out at a temperature of about -20°C to 60°C, particularly about 0°C to room temperature, for example, for 1 to 5 hours. This reaction is preferably carried out in the presence of acetic acid. The amount of acetic acid to be added may be generally about 1 to 2 mol for 1 mol of the tert-butyldimethylsilyl ether.

The benzyloxymethyl group may be removed by hydrogenolysis reaction using a catalyst such as palladium/carbon, palladium hydroxide/carbon and the like. The amount of the catalyst to be used may be generally about 5 to 20% by weight with respect to the benzyloxymethyl ether. Generally, this reaction is preferably carried out in a medium such as methanol, ethanol, tetrahydrofuran, acetic acid and the like. The amount of the medium to be used may be generally about 1 to 5 mL for 1 g of the benzyloxymethyl ether. Generally, this reaction is preferably carried out at a temperature of about -10°C to 50°C, particularly at room temperature, for example, for 3 to 10 hours.

The acetyl group may be removed by subjecting the acetate to a hydrolysis reaction using a base such as sodium carbonate, potassium carbonate, sodium hydroxide, potassium hydroxide and the like. The amount of the base to be added may be generally about 0.1 to 10 mol for 1 mol of the acetate. Generally, this reaction is preferably carried out in a medium such as methanol, ethanol, tetrahydrofuran or 1,4-dioxane, or a mixture thereof with water. The amount of the medium to be used may be generally about 1 to 5 mL for 1 g of the acetate. Generally, this reaction is preferably carried out at a temperature of about -20°C to 100°C, particularly about 0°C to 50°C, for example, for 1 to 5 hours.

In accordance with the present invention, a compound having the hydroxyl group attached to the asymmetric carbon atom *1, which is derived from the reduction of the corresponding carbonyl group, can be used in the following reactions without protection of the hydroxyl group. It is preferred, however, that the hydroxyl group is protected with a protecting group R⁵ as occasion requires before the compound is used.

The amino-protecting group may be removed by a known method indicated in literatures accepted in the art (for example, T. W. Greene, P. G. M. Wuts, et al., Protective Groups in Organic Synthesis, Wiley-Interscience Publication). For example, the benzyl group may be removed by a hydrogenolysis reaction using a catalyst such as palladium/carbon, palladium hydroxide/carbon and the like. The amount of the catalyst to be used may be generally about 5% to 20% by weight with respect to the protected amine. Generally, this reaction is preferably carried out in a medium such as methanol, ethanol, tetrahydrofuran, acetic acid and the like. The amount of the medium to be used may be generally about 1 to 50 mL for 1 g of the protected amine. Generally, this reaction is preferably carried out at a temperature of about -10°C to 50°C, particularly at room temperature, for example, for 3 to 10 hours. When R²¹ is a halogen atom, however, the amino-protecting group is removed by a known method indicated in the literatures (M. Koreeda et al., Journal of Organic Chemistry, 49, p. 2081 (1984) and S. Gubert et al., Synthesis, 4, p. 318 (1991)).

The acetyl group may be removed according to the acetate hydrolyzing process using a base as set forth above. When an acyl group is used as the amino-protecting group, however, it may be preferred that this hydrolysis reaction is generally carried out at a temperature of room temperature to about 100°C.

The tert-butoxycarbonyl (Boc) group may be removed by reacting the corresponding protected amine with a known mineral acid or Lewis acid. The known mineral acid or Lewis acid may be hydrochloric acid, hydrobromic acid, sulfuric acid, acetic acid, trifluoroacetic acid, aluminum chloride, bromotrimethylsilane, iodotrimethylsilane and the like, with hydrochloric acid being preferred. The amount of the mineral acid or Lewis acid to be added can generally vary from about the same molar amount to a solvent amount with respect to the protected amine. This reaction can be carried out in a medium. However, this reaction may also be preferably carried out using the above acid as a medium. The medium may be a lower alcohol such as methanol, ethanol, n-propanol and the like, 1,4-dioxane, tetrahydrofuran, acetonitrile, dichloromethane and the like, with methanol and ethanol being preferred. Generally, this reaction is preferably carried out at a temperature of about -30°C to 100°C, particularly about 0°C to room temperature, for example, for 1 to 10 hours.

The hydroxyl- and amino-protecting groups may be sequentially or simultaneously removed. For example, when R²¹ is a benzyloxy group, R³ is a benzyl group and R⁴ is a benzyl or benzyloxycarbonyl group, they can be removed under the same reaction condition and are preferably simultaneously removed. When R²¹ is a benzyloxy group and R⁴ is a tert-butoxycarbonyl group, they are removed by sequential steps comprising, for example, the first removal of the tert-butoxycarbonyl group as R⁴ followed by the removal of the benzyloxy group as R²¹. The sequence of the removals is not limited to the above and is preferably selected depending on the physical properties of the compound to be deprotected. The condition for removing each protecting group is as set forth above. In addition, these deprotections can be carried out with reference to the teachings of JP-A-9-249623.

Examples of a compound represented by the formula (1) include:
2-[N-[2-(9H-carbazol-2-yloxy)ethyl]amino]-1-[(3-methylsulfonylamino)phenyl]ethanol;
2-[N-[2-(9H-carbazol-2-yloxy)ethyl]amino]-1-[(4-hydroxy-3-methylsulfonylamino)phenyl]ethanol;
2-[N-[2-(9H-carbazol-2-yloxy)ethyl]amino]-1-[(4-bromo-3-methylsulfonylamino)phenyl]ethanol; and
2-[N-[2-(9H-carbazol-2-yloxy)ethyl]amino]-1-[(4-chloro-3-methylsulfonylamino)phenyl]ethanol.

The most preferred examples are the above-mentioned compounds in the R-form.

The present processes for the preparation of a compound represented by the formula (1) are illustrated in more detail in the followings.

### [Preparation Process 1]

A compound of the formula (7) is chlorinated to give a compound of the formula (6) which is reduced to give a chlorohydrin compound of the formula (5). Then, the chlorohydrin compound is treated with alkali to give an epoxy compound of the formula (4). The epoxy compound is then reacted with a compound of the general formula (8) to give a dialcohol compound of the formula (3). The primary alcohol moiety of the dialcohol compound is brominated. The resulting brominated compound is reacted with a compound represented by A'-OH to give an amino alcohol compound of the formula (2). Finally, the protecting groups of the amino alcohol compound are simultaneously or sequentially removed to give a compound of the formula (1).

A compound of the formula (7) can be synthesized by introducing an amine-protecting group R³ into 4'-R²¹-3'-methylsulfonylaminoacetophenone, which can be synthesized by a method indicated in the literatures (for example, A. A. Larsen et al., J. Med. Chem., 10, p. 462 (1967), or C. Kaiser et al., J. Med. Chem., 7, p. 49 (1974)) or in JP-A-9-249623 (WO97/25311).

A compound of the formula (6) is novel and can be obtained by chlorinating a compound of the formula (7) set forth above. The chlorinating process can be carried out using a commonly used chlorinating agent. A compound of the formula (6) can also be synthesized by a method indicated in the literatures (for example, D. Masilamani et al., J. Org. Chem., 46, p. 4486 (1981)). For example, the chlorinating agent may be sulfuryl chloride. That is, a compound of the formula (7) can be chlorinated by reacting the compound with sulfuryl chloride in the presence of methanol in an organic solvent such as methylene chloride or toluene.

A compound of the formula (5), which is a novel compound showing a relatively good crystallinity, is characteristic of the Preparation Process as an important intermediate.

A compound of the formula (5) can be obtained by reducing a compound of the formula (6) with a known reducing agent. The reducing agent may be sodium borohydride, borane, diisobutylaluminum hydride and the like. A compound of the formula (6) may be preferably reduced with a metal hydride such as sodium borohydride and the like or with hydrogen in the presence of a platinum group metal catalyst such as palladium and the like. The amount of sodium borohydride to be added may be generally about 1 to 3 mol for 1 mol of the compound of the formula (6). Generally, this reaction is preferably carried out in a lower alcohol. The lower alcohol may be methanol, i-propanol and the like, with ethanol being preferred. The amount of the lower alcohol to be used may be generally about 1 to 5 mL for 1 g of the compound of the formula (6). When the solubility of the compound is insufficient, it may be preferred that generally about 1 to 5 mL of tetrahydrofuran for 1 g of the compound of the formula (6) is added as a cosolvent. Generally, this reaction is preferably carried out at a temperature of about -20°C to 50°C, particularly about 0°C to room temperature, for example, for about 1 to 5 hours.

In addition, if an optical isomer of either R-form or S-form with respect to *1 of the formula (5) is to be obtained, it can be obtained by asymmetric reduction with a hydrogen donating compound in the presence of an asymmetric reduction catalyst known from a variety of literatures (for example, Achiwa et al. Chem. Pharm., Bull., 43, p. 748 (1995) or Noyori et al. J. Am. Chem. Soc., 118, p. 2521 (1996)).

WO97/20789 and JP-A-9-157196 each teach a variety of processes for synthesizing an optically active alcohol from a ketone compound. A catalyst for the asymmetric reduction may be prepared from a metal complex and a ligand before the asymmetrical reduction is carried out, or it may also be prepared from a metal complex and a ligand in the reaction system of the asymmetric reduction. That is, a variety of transition metals with various ligands are used as a metal complex. Most preferably, a transition metal complex is used which may be represented by MXₘLₙ wherein M represents a transition metal of the VIII group such as iron, cobalt, nickel, ruthenium, rhodium, iridium, osmium, palladium, platinum and the like; X represents a hydrogen atom, a halogen atom, a carboxyl group, a hydroxyl group, an alkoxyl group and the like; L represents a neutral ligand such as an aromatic compound, an olefin compound and the like; and, m and n represent an integer. Ruthenium is one of preferred transition metal to be contained in such transition metal complexes. When the neutral ligand is an aromatic compound, it may be a monocyclic aromatic compound. The aromatic compound may be substituted with one or more of various substituents including a hydrogen atom, a saturated or unsaturated hydrocarbon group, an allyl group, a functional group containing heteroatom(s) and the like, on any positions of the aromatic compound. The substituents may be specifically an alkyl group such as methyl, ethyl, propyl, i-propyl, butyl, tert-butyl, pentyl, hexyl, heptyl and the like; a cycloalkyl group such as cyclopropyl, cyclobutyl, cyclopentyl, cyclohexyl and the like; an unsaturated hydrocarbon group such as benzyl, vinyl, allyl and the like; or a functional group containing heteroatom(s) such as hydroxyl, alkoxyl, alkoxycarbonyl and the like.

In addition, specific examples of the metal complex include
[(S,S)-N-(p-toluenesulfonyl)-1,2-diphenylethylenediamine]benzene ruthenium complex;
[(S,S)-N-(p-toluenesulfonyl)-1,2-diphenylethylenediamine](p-cymene) ruthenium complex;
[(S,S)-N-methanesulfonyl-1,2-diphenylethylenediamine]benzene ruthenium complex;
[(S,S)-N-trifluoromethanesulfonyl-1,2-diphenylethylenediamine]mesitylene ruthenium complex;
[(S,S)-N-methanesulfonyl-1,2-diphenylethylenediamine] (p-cymene) ruthenium complex;
[(S,S)-N-benzenesulfonyl-1,2-diphenylethylenediamine](p-cymene) ruthenium complex;
[(S,S)-N-(p-fluorobenzenesulfonyl)-1,2-diphenylethylenediamine](p-cymene) ruthenium complex;
[(S,S)-N-trifluoromethanesulfonyl-1,2-diphenylethylenediamine](p-cymene) ruthenium complex;
[(S,S)-N-(p-methoxybenzenesulfonyl)-1,2-diphenylethylenediamine](p-cymene) ruthenium complex;
[(S,S)-N-methanesulfonyl-1,2-diphenylethylenediamine]mesitylene ruthenium complex;
[(S,S)-N-(p-toluenesulfonyl)-1,2-diphenylethylenediamine]mesitylene ruthenium complex;
hydride-[(S,S)-N-(p-toluenesulfonyl)-1,2-diphenylethylenediamine]benzene ruthenium complex;
hydride-[(S,S)-N-(p-toluenesulfonyl)-1,2-diphenylethylenediamine](p-cymene) ruthenium complex;
hydride-[(S,S)-N-methanesulfonyl-1,2-diphenylethylenediamine]benzene ruthenium complex;
hydride-[(S,S)-N-trifluoromethanesulfonyl-1,2-diphenylethylenediamine]mesitylene ruthenium complex;
hydride-[(S,S)-N-methanesulfonyl-1,2-diphenylethylenediamine](p-cymene) ruthenium complex;
hydride-[(S,S)-N-benzenesulfonyl-1,2-diphenylethylenediamine] (p-cymene) ruthenium complex;
hydride-[(S,S)-N-(p-fluorobenzenesulfonyl)-1,2-diphenylethylenediamine](p-cymene) ruthenium complex;
hydride-[(S,S)-N-trifluoromethanesulfonyl-1,2-diphenylethylenediamine](p-cymene) ruthenium complex;
hydride-[(S,S)-N-(p-methoxybenzenesulfonyl)-1,2-diphenylethylenediamine](p-cymene) ruthenium complex;
hydride-[(S,S)-N-methanesulfonyl-1,2-diphenylethylenediamine]mesitylene ruthenium complex;
hydride-[(S,S)-N-(p-toluenesulfonyl)-1,2-diphenylethylenediamine]mesitylene ruthenium complex;
chloro-[(S,S)-N-(p-toluenesulfonyl)-1,2-diphenylethylenediamine]benzene ruthenium complex;
chloro-[(S,S)-N-(p-toluenesulfonyl)-1,2-diphenylethylenediamine](p-cymene) ruthenium complex;
chloro-[(S,S)-N-methanesulfonyl-1,2-diphenylethylenediamine]benzene ruthenium complex;
chloro-[(S,S)-N-trifluoromethanesulfonyl-1,2-diphenylethylenediamine]mesitylene ruthenium complex;
chloro-[(S,S)-N-methanesulfonyl-1,2-diphenylethylenediamine](p-cymene) ruthenium complex;
chloro-[(S,S)-N-benzenesulfonyl-1,2-diphenylethylenediamine](p-cymene) ruthenium complex;
chloro-[(S,S)-N-(p-fluorobenzenesulfonyl)-1,2-diphenylethylenediamine](p-cymene) ruthenium complex;
chloro-[(S,S)-N-trifluoromethanesulfonyl-1,2-diphenylethylenediamine](p-cymene) ruthenium complex;
chloro-[(S,S)-N-(p-methoxybenzenesulfonyl)-1,2-diphenylethylenediamine](p-cymene) ruthenium complex;
chloro-[(S,S)-N-methanesulfonyl-1,2-diphenylethylenediamine]mesitylene ruthenium complex;
chloro-[(S,S)-N-(p-toluenesulfonyl)-1,2-diphenylethylenediamine]mesitylene ruthenium complex and the like.

Each of the abovementioned metal complexes can be used as it is as a catalyst for the present invention.

Further, an asymmetric reduction using a catalyst obtained by the reaction of the following rhodium complex with the following chiral phosphine ligand is known. For example, [Rh(nbd)₂]ClO₄ (wherein nbd means norbornadiene), [Rh(nbd)Cl]₂, [Rh(cod)Cl]₂ (wherein cod means cycloocta-1,5-diene), and the like are known as a rhodium complex. Examples of chiral phosphine ligand include (2R,3R)-2,3-bis(diphenylphosphino)-bicyclo[2,2,1]hept-5-ene [abbr.: (R,R)-NORPHOS]; (R)-5,5'-dimethoxy-4,4',6,6'-tetramethyl-2-diphenylphosphino-2'-dicyclohexylphosphino-1,1'-biphenyl [abbr.: (R)-MOC-BIMOP]; (R)-5,5'-dimethoxy-4,4',6,6'-tetramethyl-2,2'-bis(dicyclohexylphosphino)-1,1'-biphenyl [abbr.: (R)-Cy-BIMOP]; (2S,3S)-1,4-bis[bis(4-methoxy-3,5-dimethylphenyl)phosphino]-2,3-O-isopropylidene-2,3-butanediol [abbr.: (S,S)-MOD-DIOP]; (2S,3S)-1,4-bis(diphenylphosphino)-2,3-O-isopropylidene-2,3-butanediol [abbr.: (S,S)-DIOP]; (2S,3S)-1-diphenylphosphino-4-dicyclohexylphosphino-2,3-O-isopropylidene-2,3-butanediol [abbr.: (S,S)-DIOCP]; (R)-1-[(S)-1',2-bis(diphenylphosphino)ferrocenyl]ethanol [abbr.: (R)-(S)-BPPFOH]; (S)-1-[(S)-1',2-bis(diphenylphosphino)ferrocenyl]ethanol [abbr.: (S)-(S)-BPPFOH]; (1S,2S)-1-(diphenylphosphino)-2-[(diphenylphosphino)methyl]cyclopentane [abbr.: (S,S)-PPCP]; and (1R,2R)-1-(dicyclohexylphosphino)-2-[(diphenylphosphino)methyl]cyclopentane [abbr.: (R,R)-CPCP].

In addition, the literature (H. C. Brown et al. J. Org. Chem., 54, p. 1577 (1989)) teaches that chlorodiisopinocampheylborane may also be used for an asymmetric reduction catalyst.

When an asymmetric reduction according to the present invention is carried out in the presence of such a known catalyst, a suitable catalyst can be previously selected by checking the fact that the reduction can appropriately proceed in the presence of the catalyst. However, such a checking may limit suitable catalysts to be selected. Particularly preferred examples of the catalyst include those represented by the formula (13): 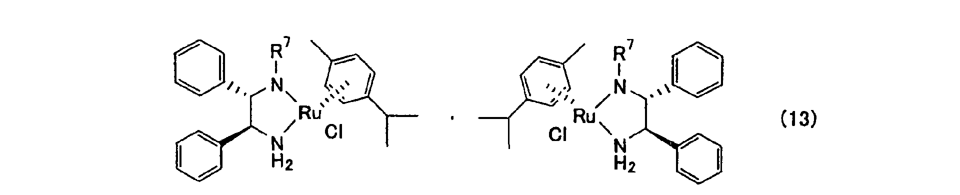 wherein R⁷ represents p-CH₃C₆H₄SO₂ or CH₃SO₂, which is obtained by reacting a ruthenium complex represented by the formula [RuCl₂(p-cymene)]₂ with a chiral ethylenediamine ligand represented by the formula (12): 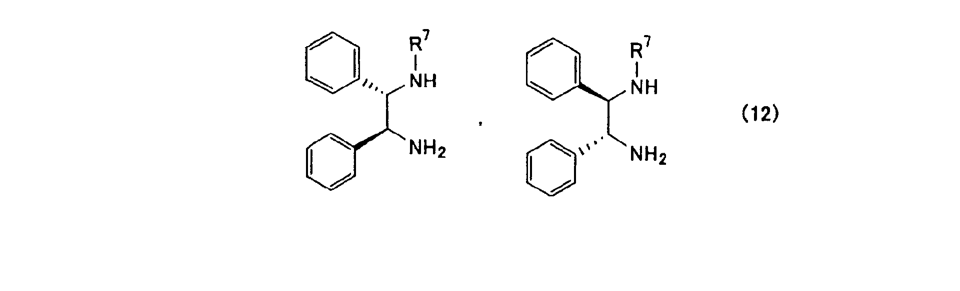 wherein R⁷ is as defined above. That is, an optically active compound (chlorohydrin) of the formula (5) can be obtained by reducing a compound of the formula (6) with hydrogen in the presence of the abovementioned ruthenium complex (13). These reactions may be carried out according to the teachings of the literature (Noyori et al., J. Am. Chem. Soc., 118, p 2521 (1996)).

An asymmetric reduction of a compound of the formula (6) with (diphenylethylenediamine)ruthenium complex can be carried out by reacting the compound with a hydrogen donating compound in the presence of the catalyst. The amount of the catalyst to be added may be generally 0.005 to 1 equivalent with respect to the compound of the formula (6). Examples of the hydrogen donating compound include hydrogen gas, alcohol compounds such as methanol, ethanol, n-propanol, i-propanol and the like, formic acid and salts thereof, amine compounds such as triethylamine and the like, unsaturated hydrocarbons having partially saturated carbon linkages such as tetralin, decalin and the like, heterocyclic compounds, hydroquinon, phosphorous acid and the like. The reaction is preferably carried out in a reaction solvent. Examples of the reaction solvent include aromatic solvents such as toluene, xylene and the like, halogen-containing solvents such as dichloromethane, chloroform and the like, polar solvents such as N,N-dimethylformamide, N,N-dimethylacetamide and the like, sulfoxide solvents such as dimethylsulfoxide, sulfolane and the like, nitrile solvents such as acetonitrile and the like, ether solvents such as diethyl ether, tetrahydrofuran, 1,4-dioxane and the like. The reaction solvent is preferably an ether solvent such as tetrahydrofuran and the like. The reaction solvent preferably function as the abovementioned hydrogen donor, too. The amount of the reaction solvent may be 0.1% to 100% by weight with respect to the compound of the formula (6).

In addition, the reaction is preferably carried out in the presence of a base. The base may be potassium hydroxide, sodium hydroxide, lithium hydroxide, potassium methoxide, potassium tert-butoxide and the like, with potassium hydroxide, sodium hydroxide or lithium hydroxide being preferred. The reaction is carried out at a temperature of about -30°C to 50°C, preferably at a temperature of about -20°C to 10°C with good optical yield. The reaction time is 0.5 to 10 days, preferably 1 to 3 days.

An asymmetric reduction with chlorodiisopinocampheylborane can be carried out by reducing a compound of the formula (6) with diisopinocampheylchloroborane. The amount of the reducing agent to be used is 1.0 to 10.0 equivalents, preferably 1.0 to 3.0 equivalents with respect to the compound of the formula (6). Examples of the reaction solvent include aromatic solvents such as toluene, xylene and the like, ether solvents such as diethyl ether, tetrahydrofuran, 1,4-dioxane and the like, halogen-containing solvents such as dichloromethane, chloroform and the like, and saturated aliphatic solvents such as pentane, hexane and the like. An ether solvent such as tetrahydrofuran and the like is preferably used. The reaction is carried out at a temperature of about - 50°C to 50°C, preferably at a lower temperature of about -20°C to 0°C with good optical yield. The reaction time is 1 to 24 hours, preferably 5 to 15 hours.

A compound of the formula (4) is a useful intermediate, which is a novel compound showing a good crystallinity. The compound can be purified by recrystallization and is further useful for improving the optical purity of the resulting compound. A compound of the formula (4) can be synthesized from a compound of the formula (5) by a conventional process. For example, such a process may comprises reacting a compound of the formula (5) with 1- to 5-fold moles of alkali in an alcohol solvent such as methanol, ethanol and the like or acetone at a temperature of room temperature to the reflux temperature of the solvent used. The alkali may be sodium carbonate, potassium carbonate, sodium hydroxide, potassium hydroxide and the like.

The reaction of a compound of the formula (4) with a compound of the formula (8) can be carried out in a common medium, for example in an organic solvent such as dimethylsulfoxide, a linear or cyclic ether, dimethylformamide, dimethylacetamide, alcohol compounds and the like.

Though a compound of the formula (4) and a compound of the formula (8) are usually used in the equimolar amount, the latter is preferably used in an excess amount. The reaction temperature can be suitably selected and may be generally a temperature of room temperature to the reflux temperature of the solvent used. The reaction time can be suitably selected depending on the reaction conditions and may be generally selected such that the reaction can provide the maximum yield.

In addition, there is a report (Tetrahedron Letters, 27, p. 2451 (1986)) that the addition of trimethylsilylacetamide (TMSA), [N,O-bis(trimethylsilyl)acetamide], hexamethyldisilazane (HMDS) or bis(trimethylsilyl)urea to the reaction can shorten the reaction time and improve the yield. This may be suitably applied to the present reaction.

A compound of the formula (8) can be prepared according to the reaction condition of the Referential Example. A compound of the formula (8) in which R⁴ is a benzyl group is particularly preferred, since a commercially available product (mfd. by TOKYO KASEI KOGYO) can be used.

The side chain hydroxyl group (primary hydroxyl group) of a compound of the formula (3) can be converted into a bromine atom by brominating the compound with a known brominating agent such as hydrogen bromide/acetic acid, phosphorus tribromide, phosphorus pentabromide, thionyl bromide, bromine/triphenylphosphine, carbon tetrabromide/triphenylphosphine, N-bromosuccinimide/triphenylphosphine and the like. For example, about 1 to 10 mol of phosphorus tribromide for 1 mol of a compound of the formula (3) may be reacted. Generally, this reaction is preferably carried out in an inert medium. The inert medium may be 1,2-dichloroethane, carbon tetrachloride and the like, with dichloromethane being preferred. The amount of the inert medium to be used may be generally 1 to 10 mL for 1 g of a compound of the formula (3). Generally this reaction is preferably carried out at a temperature of about -30°C to 100°C, particularly about 0°C to 50°C, for example, for 1 to 5 hours.

Generally, the condensation reaction with a compound represented by A'-OH is preferably carried out by reacting 1- to 5-fold moles of the compound represented by A'-OH with the compound of the formula (3) in a basic condition. The basic condition is preferably adjusted with a metal alkoxide obtained from an alkali such as potassium carbonate, potassium hydroxide, sodium hydroxide, sodium hydride, potassium hydride, potassium tert-butoxide and the like. The amount of the metal alkoxide to be used may be generally about 1 to 3 mol for 1 mol of the compound of the formula (3). Generally, this reaction is preferably carried out in an inert medium. The inert medium may be acetone, 2-butanone, tetrahydrofuran, N,N-dimethylacetamide, dimethylsulfoxide, sulfolane and the like, with N,N-dimethylformamide being preferred. The amount of the inert medium to be used may be 1 to 10 mL for 1 g of the brominated compound. Generally, this reaction is preferably carried out at a temperature of room temperature to about 100°C, for example, for 3 to 10 hours.

Next, the simultaneous or sequential removals of the protecting groups according to the method set forth above can give a compound of the formula (1).

In each step of the synthesizing route set forth above, the produced material is preferably purified by a known purifying means, such as column chromatography and the like. However, a novel compound of the formula (5) or (4) is relatively good in crystallinity and can be used in the following reaction step after being subjected to a simple recrystallizing treatment without complicated processes. Therefore, the present process, which can save cost and labor, is a preferred process. In addition, the present process is also preferred in that each reaction step results in good yield.

### [Preparation Process 2]

A compound of the formula (2) set forth above can be synthesized by the following process. That is, a compound of the formula (2) can be synthesized by one reaction step comprising condensing a compound of the formula (4) with a compound of the formula (9).

The condensation reaction of a compound of the formula (4) with a compound of the formula (9) can be carried out according to the reaction condition for reacting a compound of the formula (4) with a compound of the formula (8) in Preparation Process 1 set forth above. R³ of the formula (4) and R⁴ of the formula (9) are the same in that they are an amino-protecting group. However, they do not necessarily represent the same substituent and may be different from each other.

A compound of the formula (9) can be obtained by protecting a known primary amine compound (NH₂-CH₂CH₂-OA') with a protecting group R⁴, wherein the amine compound may be synthesized according to the process disclosed in JP-A-9-249623. That is, a benzyl group as R⁴ may be introduced by reductive alkylation with benzaldehyde or alkylation with benzyl halide, benzyl sulfonate and the like. The amount of benzaldehyde to be added according to such a reductive alkylation reaction may be generally about 1 to 1.5 mol for 1 mol of the primary amine. Generally, this reaction is preferably carried out in a medium such as tetrahydrofuran, water, methanol, ethanol and the like. Methanol may be particularly preferred as the medium. The amount of the medium to be used may be generally about 10 to 100 mL for 1 g of the primary amine. Generally, this reaction is preferably carried out at room temperature, for example, for 3 to 10 hours. Generally, this reaction is preferably carried out in the presence of a platinum group metal catalyst. The platinum group metal catalyst may be preferably platinum oxide. The amount of the platinum group metal catalyst to be used generally about 0.01 to 0.1 mol for 1 mol of the primary amine. This reaction is carried out under a hydrogen atmosphere. Generally, the pressure of hydrogen may be preferably 1 to 10 atm, particularly 1 to 3 atm.

Alternatively, a compound of the formula (9) may be synthesized from a compound represented by A'-OH by a two step process. That is, the compound may be obtained by reacting a known compound represented by A'-OH with 1,2-dibromoethane to give a compound of the formula (11): 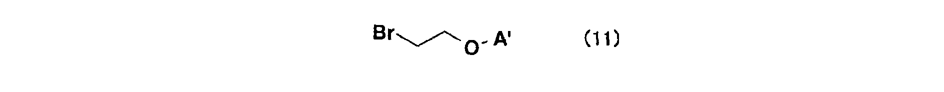 and reacting the thus obtained compound with an amine (NH₂-R⁴)(wherein R⁴ is an substituted benzyl group).

The reaction of a compound represented by A'-OH with 1,2-dibromoethane may be generally carried out in an organic solvent in the presence of a base at a temperature of room temperature to the reflux temperature of the solvent selected. The amount of 1,2-dibromoethane to be used is preferably 3 to 15 mol for 1 mol of the compound represented by A'-OH. The solvent may be dimethylformamide, dimethylacetamide, 2-butanone, acetonitrile, diglyme, tetrahydrofuran and the like. The base may be potassium carbonate, sodium carbonate, sodium hydroxide, potassium hydroxide, triethylamine, pyridine, sodium hydride, sodium methoxide and the like. The amount of the base to be used is preferably 1 to 5 mol for 1 mol of the compound represented by A'-OH. The amount of the medium to be used may be generally about 5 to 100 mL for 1 g of the compound represented by A'-OH. Generally, this reaction is preferably carried out at a temperature of 60°C to 90°C, for example, for 3 to 24 hours.

The reaction of a compound of the formula (11) with a compound represented by NH₂-R⁴ may be carried out in a solvent or without solvent at a temperature of 60°C to 100°C. The amount of the compound represented by NH₂-R⁴ to be used may be 2 to 10 mol for 1 mol of the compound of the formula (11). The solvent may be dimethylformamide, dimethylacetamide, dimethylsulfoxide, 2-propanol and the like.

A compound represented by A'-OH can be prepared according to the processes disclosed in JP-A-9-249623 (WO97/25311) and WO99/01431. However, for example, 2-hydroxycarbazole is commercially available (mfd. by Aldrich), and using such a commercially available product is convenient and therefore preferred.

The simultaneous or sequential removals of the protecting groups of the thus obtained compound of the formula (2) in the same way as Preparation Process 1 can give a compound of the formula (1).

A compound of the formula (9), which can be synthesized from a compound represented by A'-OH by two step process according to the synthesizing route set forth above, is good in crystallinity and can be obtained by only filtration treatment without complicated process. The said compound can also be used for the reaction with a compound of the formula (4). Therefore, the synthesizing route is a preferred process which can save cost and labor. In addition, the present process is also preferred in that each reaction step is results in good yield and that the number of reaction steps is relatively few.

### [Preparation Process 3]

A compound of the formula (1) can also be obtained by the following process. That is, the process comprises protecting the hydroxyl group of a compound of the formula (5) with a protecting group R⁵, or halogenating a compound of the formula (5) followed by protecting the hydroxyl group with a protecting group R⁵, to give a compound of the formula (10); reacting the compound of the formula (10) with a compound of the formula (8) to give an alcohol compound of the formula (3a); brominating the primary alcohol of the resulting alcohol compound, followed by the reaction with a compound represented by the above A'-OH to give an amino alcohol compound of the formula (2a); and simultaneously or sequentially removing the protecting groups of the thus obtained compound of the formula (2a) to give a compound of the formula (1).

The hydroxyl group of a compound of the formula (5) can be protected by the method set forth above.

When the replacement of chlorine atom of a compound of the formula (5) (chlorinated compound) with bromine or iodine atom is needed, such a compound of the formula (5) may be synthesized using a commonly used brominating or iodinating agent. The brominating or iodinating agent is not limited as long as it can brominate or iodinate the compound and may be, for example, sodium bromide or iodide. Specifically, a compound of the formula (10) may be obtained by heating a compound of the formula (5) with 3 to 10 mol of sodium bromide or iodide for 1 mol of the chlorinated compound in a solvent such as acetone and the like at the reflux temperature for 3 to 72 hours, followed by protecting the hydroxyl group with a protecting group R⁵ by the method for protecting a hydroxyl group set forth above.

The coupling reaction of a compound of the formula (10) with an amine compound of the formula (8) comprises heating the halide of the formula (10) with 1- to 3-fold moles of the amine compound of the formula (8) in a polar solvent such as dimethylformamide, dimethylacetamide, dimethylsulfoxide, 2-propanol and the like in the absence or presence of an amine such as triethylamine, diisopropylethylamine and the like as a proton-trapping agent at a temperature of room temperature to 90°C, preferably at 80°C for 5 to 24 hours.

The bromination of a compound of the formula (3a) and the successive condensation reaction with a compound represented by A'-OH can be carried out according to the process set forth above (Preparation Process 1) for synthesizing a compound of the formula (2) from a compound of the formula (3).

A compound of the formula (1) can be obtained by deprotecting the thus obtained compound of the formula (2) according to Preparation Process 1 set forth above, preferably by the sequential removals of first R⁵ and next the protecting group of the formula (2).

In each step of the synthesizing route set forth above, the product obtained is preferably purified by a known purifying means, such as column chromatography and the like. The present process is preferred in that each reaction step results in good yield and that the number of reaction steps is relatively few.

### [Preparation Process 4]

A compound of the formula (1) can also be synthesized by the following process. That is, a compound of the formula (1) can be obtained by reacting a compound of the formula (10) set forth above with an amine compound of the formula (9) set forth above, and simultaneously or sequentially removing the protecting groups of the thus obtained compound of the formula (2a).

The coupling reaction of a compound of the formula (10) with an amine compound of the formula (9) can be carried out according to the reaction of a compound of the formula (10) with a compound of the formula (8) of Preparation Process 3.

A compound of the formula (9), which can be synthesized from a compound represented by A'-OH by a two step process according to the synthesizing route set forth above, is good in crystallinity and can be obtained by only filtration treatment without complicated processes. The compound can also be used for the reaction with a compound of the formula (10). Therefore, the synthesizing route is a preferred process which can save cost and labor. In addition, the present process is also preferred in that each reaction step is results in good yield and that the number of reaction steps is relatively few.

The step of asymmetricreduction of the carbonyl group of a compound represented by the formula (6), which compound is a common intermediate of Preparation Processes 1 to 4, is characteristic of the processes, and the thus reduced compound is a useful intermediate.

As set forth above, a compound of the formula (1) can exist in the two different optically active substances. The processes described herein can provide a racemic mixture and also an optically active substance as occasion requires. The reactions set forth above should not alter the relating stereochemistry.

When a mixture of two optical isomers is obtained, the mixture can be resolved into optical isomers thereof as their acid addition salts with an optically active acid such as camphorsulfonic acid, mandelic acid or substituted mandelic acid by a suitable method such as fractional crystallization. Such a fractional crystallization may be carried out using a suitable solvent, preferably a lower alcohol, such as methanol, ethanol, i-propanol or a mixture thereof. Each pair of enantiomers can be resolved into pure isomers by formation of diastereomeric salt, chromatography using an optically active column, or other means. When one of the starting materials is optically active, the thus obtained mixture of diastereomers can be resolved into pure isomers by the above-mentioned means. This resolution may be applied to a compound of the formula (1) and also applied to an intermediate amino alcohol represented by the formula (2), (2a), (3) or (3a) which is obtained in a step of the preparation processes set forth above (Preparation Processes 1 to 4). Optical resolution and purification of the present compounds can provide a preferred pharmaceuticals which comprises a single isomer having higher activities and thereby has improved efficacy without side effect.

Salts of compounds of the formulae (1), (2), (2a), (3) and (3a) according to the present invention may be a known salt, and examples thereof include hydrochloride, hydrobromide, sulfate, hydrogensulfate, dihydrogen phosphate, citrate, maleate, tartrate, fumarate, gluconate and methanesulfonate, and acid addition salts with an optically active acid such as camphorsulfonic acid, mandelic acid or substituted mandelic acid. Among them, pharmaceutically acceptable salts are particularly preferred. When a compound of the formula (1), (2), (2a), (3) or (3a) is converted into its salt, an acid addition salt of the compound can be obtained by dissolving the compound in an alcohol such as methanol, ethanol and the like, to which the equivalent amount to several times amount of the acid is then added. The acid to be used may be a pharmaceutically acceptable mineral or organic acid, such as hydrochloric acid, hydrobromic acid, sulfuric acid, hydrogensulfate, dihydrogen phosphate, citric acid, maleic acid, tartaric acid, fumaric acid, gluconic acid, methanesulfonic acid and the like.

### Examples

The following examples further illustrate this invention but are not intended to limit it in any way.

The thin layer chromatography (TLC) used was Precoated silica gel 60 F254 (mfd. by MERCK). The detecting process was carried out with UV (254 nm) irradiation and coloration with ninhydrin after development with chloroform/methanol (1:0 to 4:1), chloroform/acetone (1:0 to 10:1) or n-hexane/ethyl acetate (1:0 to 1:10). R_{f} values of TLC were obtained on free amines. The organic layers were dried over anhydrous magnesium sulfate or anhydrous sodium sulfate. The column chromatography process was carried out on silica gel 60 (230-400 mesh; mfd. by MERCK). The determination of nuclear magnetic resonance spectrum (NMR) was carried out using Gemini-300 (FT-NMR; mfd. by Varian). Mass spectrum (MS) was determined by the fast atom bombardment mass spectrometry (FAB-MS) with JEOL-JMS-SX102. The melting point was determined with Melting-Point Apparatus BY-2 (mfd. by YAZAWA KAGAKU).

### [Referential Example]

### Synthesis of N-benzyl-N-[2-(9H-carbazol-2-yloxy)ethyl]amine (Compound 9)

Benzaldehyde (9.38 g) was added to a solution of N-[2-(9H-carbazol-2-yloxy)ethyl]amine (20 g; synthesized according to the process indicated in JP-A-9-249623) in methanol (500 mL). The mixture was stirred at room temperature for 1 hour. Platinum oxide (1.00 g; mfd. by Wako Pure Chemical Industries) was added under an argon atmosphere and the mixture was then stirred under a hydrogen atmosphere at atmospheric pressure for 3 hours. After the atmosphere in the reaction system was replaced with argon, benzaldehyde (1.88 g) was added. The mixture was further stirred under a hydrogen atmosphere at atmospheric pressure for 3 hours. After the atmosphere was replaced with argon, the catalyst was filtered off and the solvent was distilled off under reduced pressure. The residue was recrystallized from ethanol and dried under reduced pressure to give the title compound (25.2 g) as a slightly yellowish crystal.
R_{f}=0.6 (10:1 chloroform/methanol);
MS: 317 (MH⁺);
¹H-NMR (DMSO-d₆; free form): 2.30 (1H, s), 2.91 (2H, t, J=5.8), 3.79 (2H, s), 4.11 (2H, t, J=5.8), 6.77 (1H, dd, J=8.5, 2.2), 6.96 (1H, d, J=2.2), 7.10 (1H, m), 7.20-7.44 (7H, m), 7.92-8.00 (2H, m), 11.09 (1H, s).

### [Intermediate]

### Alternative synthesis of N-benzyl-N-[2-(9H-carbazol-2-yloxy)ethyl]amine (Compound 9)

### Step A. Synthesis of 2-(2-bromoethoxy)carbazole

A mixture of 2-hydroxycarbazole (30 g; mfd. by Aldrich), potassium carbonate (113.1 g; mfd. by KANTO CHEMICAL) and 1,2-dibromoethane (211 mL; mfd. by TOKYO KASEI KOGYO) in 2-butanone (165 mL; mfd. by Wako Pure Chemical Industries) was stirred vigorously at reflux temperature for 28 hours. The reaction solution was poured into water (1050 mL) all at once and stirred. The crystal precipitated was separated by filtration, washed with water (1 L) and 2-propanol (250 mL), and then dried under reduced pressure at room temperature to give the title compound (43.43 g) as a white solid.
R_{f}=0.51 (1:2 ethyl acetate/n-hexane);
¹H-NMR (DMSO-d₆): 3.82-3.85 (2H, m), 4.36-4.43 (2H, m), 6.80 (1H, dd, J=8.5, 2.2), 6.99 (1H, d, J=2.2), 7.11 (1H, m), 7.29 (1H, m), 7.42 (1H, d, J=8.3), 7.98 (1H, d, J=8.5), 8.00 (1H, d, J=7.7), 11.13 (1H, s).

### Step B. Synthesis of N-benzyl-N-[2-(9H-carbazol-2-yloxy)ethyl]amine

A mixture of the compound (80 g; obtained in the above Step A) in benzyl amine (270 mL; mfd. by TOKYO KASEI KOGYO) was stirred with heating at an internal temperature of 100°C for 4 hours. The reaction solution was poured into water (2.3 L) all at once and stirred for 30 minutes. The crystal precipitated was separated by filtration, washed with water (1.5 L) and 2-propanol (1 L), and then dried under reduced pressure at room temperature to give the title compound (76.5 g).
R_{f}=0.33 (1:2 ethyl acetate/n-hexane).

The thus obtained compound showed the same NMR data with those of the compound of Referential Example.

### Example 1

### Synthesis of 2-chloro-1-[3-(N-benzyl-N-methylsulfonylamino)phenyl]ethanone (Compound 6; Preparation Process 1)

### Step A. Synthesis of 3'-(N-benzyl-N-methylsulfonylamino)acetophenone

To a solution of 3'-(methylsulfonylamino)acetophenone (227 g; prepared by the process reported by A. A. Larsen et al., J. Med. Chem., 10, p. 462 (1967) or C. Kaiser et al., L Med. Chem., 7, p. 49 (1974), or by the process indicated in JP-A-9-249623 (WO97/25311)) in dimethylformamide (2.14 L) were added potassium carbonate (884 g; mfd. by Wako Pure Chemical Industries), benzyl bromide (254 mL; mfd. by Wako Pure Chemical Industries) and sodium iodide (176 g; mfd. by Wako Pure Chemical Industries). The mixture was stirred at room temperature for 12.2 hours. The reaction solution was poured into water (10 L) and stirred for 1 hour. The brown solid precipitated was separated by filtration and dissolved in ethyl acetate (2 L). The resulting solution was concentrated under reduced pressure and the residue was dissolved in hot toluene. The insoluble matter was then filtered off to give the filtrate 1. The aqueous layer was extracted with ethyl acetate (8 L), dried, concentrated and combined with the filtrate 1. The combined filtrate was concentrated and crystallized from toluene (500 mL) and heptane (150 mL). The solid precipitated was separated by filtration, washed with heptane (300 mL × 3) and then dried under reduced pressure at room temperature to give the title compound (281 g) as a light yellow solid.
R_{f}=0.32 (1:1 ethyl acetate/n-hexane);
¹H-NMR (CDCl₃): 2.55 (3H, s), 2.98 (3H, s), 4.89 (2H, s), 7.24-7.27 (5H, m), 7.38-7.48 (2H, m), 7.82-7.86 (2H, m).

### Step B.Synthesis of 2-chloro-1-[3-(N-benzyl-N-methylsulfonylamino)phenyl]ethanone

A solution of sulfuryl chloride (0.46 mL; mfd. by Wako Pure Chemical Industries) in dichloromethane (3.3 mL) was added dropwise to a solution of the compound (1 g; obtained in the above step A) in dichloromethane (1.65 mL) and methanol (0.53 mL) over 1 hour. After the reaction was completed, water (10 mL) was added and the reaction solution was separated. The organic layer was washed with a 0.1 N NaOH aqueous solution (10 mL × 3), dried and concentrated under reduced pressure. The residue was purified by silica gel chromatography (1:2 ethyl acetate/hexane) to give the title compound (987 mg) as a colorless solid.
R_{f}=0.48 (1:1 ethyl acetate/n-hexane);
¹H-NMR (CDCl₃): 2.99 (3H, s), 4.62 (2H, s), 4.89 (2H, s), 7.21-7.31 (5H, m), 7.43-7.54 (2H, m), 7.82-7.85 (2H, m).

### Example 2

### Synthesis of (R)-2-chloro-1-[3-(N-benzyl-N-methylsulfonylamino)phenyl]ethanol (Compound 5; Preparation Process 1)

[(S,S)-N-(p-toluenesulfonyl)-1,2-diphenylethylenediamine](p-cymene) ruthenium complex (63.6 mg; synthesized according to the method reported by Noyori et al., J. Am. Chem. Soc., 118, p. 2521 (1996)) was added to a solution of 2-chloro-1-[3-(N-benzyl-N-methylsulfonylamino)phenyl]ethanone (3.38 g; synthesized in Example 1) in a formic acid/triethylamine solution (5 mL; 5:2 formic acid/triethylamine complex; mfd. by FLUKA) and tetrahydrofuran (5 mL). The mixture was stirred at room temperature for 4 hours.

After the reaction was completed, the reaction solution was added to ethyl acetate (30 mL) and water (30 mL), stirred vigorously, separated, washed with saturated brine (30 mL), and then dried. After the solvent was distilled off under reduced pressure, the residue was purified by silica gel chromatography (1:3 ethyl acetate/n-hexane) and concentrated to give the title compound (3.51 g).
R_{f}=0.40 (1:1 ethyl acetate/n-hexane);
¹H-NMR (CDCl₃): 2.77 (1H, d, J=3.3), 2.95 (3H, s), 3.52 (1H, dd, J=11.3, 8.2), 3.64 (1H, dd, J=11.3, 3.5), 4.82-4.86 (3H, m), 7.20-7.36 (9H, m).
HPLC: Retention Time (R-form: 62.9 min (S-form: 67.7 min))
Column:CHIRALCEL OD-RH (mfd. by DAICEL CHEMICAL INDUSTRIES; 4.6 mm ID × 150 mm);
Solvent: 75:25 0.1 M KPF₆/acetonitrile;
Flow rate: 0.5 mL/min;
Detecting wave length: 254 nm;
Temperature: 40°C.

### Example 3

### Synthesis of (R)-1-[3-(N-benzyl-N-methylsulfonylamino)phenyl]oxirane (Compound 4; Preparation Process 1)

Potassium carbonate (1.23 g) was added to a solution of the compound (1.52 g; obtained in Example 2) in acetone (15.2 mL). The mixture was stirred at reflux temperature for 5 hours and cooled to room temperature. Ethyl acetate (50 mL) and water (50 mL) were then added and the reaction solution was separated. The aqueous layer was extracted with ethyl acetate. The extract combined with the organic layer was dried and the solvent was distilled off under reduced pressure. The residue was purified by silica gel chromatography (1:3 ethyl acetate/n-hexane) and concentrated to give the title compound (700 mg), which was recrystallized from methanol.
m.p. 83.5-84.5°C;
R_{f}=0.47 (1:1 ethyl acetate/n-hexane);
MS: 304 (MH⁺);
¹H-NMR (CDCl₃): 2.67 (1H, dd, J=5.5, 2.5), 2.94 (3H, s), 3.12 (1H, dd, J=5.5, 4.1), 3.80 (1H, dd, J=4.1, 2.5), 4.79-4.90 (2H, m), 7.16-7.32 (9H, m).
HPLC: Retention Time (R-form: 92.9 min (S-form: 100.1 min))
Column: CHIRALCEL OB-H (mfd. by DAICEL CHEMICAL INDUSTRIES; 4.6 mm ID × 250 mm);
Solvent: 9:1 hexane/ethanol;
Flow rate: 0.5 mL/min;
Detecting wave length: 254 nm;
Temperature: 40°C.

### Example 4

### Synthesis of (R)-2-[N'-benzyl-N'-(2-hydroxyethyl)amino]-1-[3-(N-benzyl-N methylsulfonylamino)phenyl]ethanol (Compound 3; Preparation Process 1)

N-benzylethanolamine (4.5 mL; mfd. by TOKYO KASEI KOGYO) was added to the compound (6.4 g; obtained in Example 3). The mixture was stirred with heating at 100°C for 13 hours and then cooled down. The mixture was purified by silica gel chromatography (2:98 methanol/chloroform) and concentrated under reduced pressure to give the title compound (9 g) as a white amorphous solid.
R_{f}=0.35 (1:19 methanol/chloroform);
MS: 456(MH⁺);
¹H-NMR (CDCl₃): 2.55 (1H, dd, J=13.5, 9.3), 2.61-2.71 (2H, m), 2.76-2.86 (1H, m), 2.91 (3H, s), 3.54-3.72 (3H ,m), 3.83 (1H, d, J=13.5), 4.61 (1H, dd, J=9.3, 3.9), 4.82 (2H, s), 7.10-7.37 (14H, m).

### Example 5

### Synthesis of (R)-2-[N'-benzyl-N'-[2-(9H-carbazol-2-yloxy)ethyl]amino]-1-[3-(N-benzyl-N-methylsulfonylamino)phenyl]ethanol (Compound 2; Preparation Process 1)

N-bromosuccinimide (1.57 g; mfd. by Wako Pure Chemical Industries) was added to a solution of the compound (4 g; obtained in Example 4) and triphenylphosphine (2.31 g; mfd. by Wako Pure Chemical Industries) in anhydrous dichloromethane (58.6 g) at -15°C. The mixture was stirred for 15 minuets (the intermediate brominated compound: R_{f}=0.91 (1:9 methanol/chloroform)). After tetrahydrofuran (44 g), 2-hydroxycarbazole (1.61 g; mfd. by Aldrich) and 2 N NaOH (9.5 g) were added, the reaction solution was stirred at room temperature for 5 hours, concentrated, and dissolved in ethyl acetate (250 mL). The reaction solution was washed with 2 N NaOH (250 mL × 2) and then saturated brine. The organic layer was dried and the solvent was distilled off under reduced pressure. The residue was purified by silica gel column chromatography (1:10 to 1:1 ethyl acetate/n-hexane) to give the title compound (2.45 g) as a light yellow amorphous solid.
R_{f}=0.26 (1:1 ethyl acetate/n-hexane);
¹H-NMR (DMSO-d₆): 2.65-2.72 (2H, m), 2.91 (2H, m), 3.05 (3H, s), 3.76 (2H, br.s), 3.95-4.03 (2H, m), 4.65-4.73 (1H, m), 4.82 (2H, s), 5.15 (1H, d, J=3.6), 6.72 (1H, dd, J=8.5, 2.2), 6.90 (1H, d, J=2.2), 7.08-7.37 (16H, m), 7.42 (1H, d, J=8.0), 7.95 (1H, d, J=8.5), 7.99 (1H, d, J=7.7), 11.08 (1H, s).

### Example 6

### Synthesis of (R)-2-[2-(9H-carbazol-2-yloxy)ethylamino]-1-[3-(methylsulfonylamino)phenyl]ethanol hydrochloride (Compound 1; Preparation Process 1)

The compound (10 g; obtained in Example 5) was dissolved in a mixed solvent of tetrahydrofuran (100 mL) and methanol (100 mL), to which palladium hydroxide/carbon catalyst (1 g; mfd. by nacalai tesque) was added under nitrogen atmosphere. The mixture was stirred overnight under hydrogen atmosphere at atmospheric pressure at room temperature. The catalyst was filtered off and the solvent was distilled off under reduced pressure. The crystal was separated by suction filtration and washed with a mixed solvent of tetrahydrofuran and methanol (1:1). The crystal was dissolved in methanol (150 mL) and converted into its hydrochloride with 0.1 N hydrochloric acid/ethanol. The crystal precipitated was separated by suction filtration and dried under reduced pressure with heating (40°C) to give the title compound as hydrochloride (7.50 g) as a white crystal.
R_{f}=0.3 (10:1 chloroform/methanol);
MS: 441 (MH⁺).

The thus obtained compound was shown to be identical to the title compound prepared according to the known method (JP-A-9-249623) by the fact that they had the same retention time in HPLC measurements.

### Example 7

### Synthesis of (±)-2-chloro-1-[3-(N-benzyl-N-methylsulfonylamino)phenyl]ethanol (Compound 5; Preparation Process 1)

Sodium borohydride (15.7 mg; mfd. by Wako Pure Chemical Industries) was added to a solution of the compound (100 mg; obtained in Example 1) in tetrahydrofuran (0.5 mL) and methanol (1 mL) with ice cooling. The mixture was allowed to warm to room temperature with stirring. After the addition of 1 N HCl (0.41 mL), the reaction solution was concentrated and separated with ethyl acetate and water. The organic layer was dried and concentrated under reduced pressure to give the title compound.

### Example 8

### Synthesis of (±)-1-[3-(N-benzyl-N-methylsulfonylamino)phenyl]oxirane (Compound 4; Preparation Process 1)

The title compound was obtained from the compound (1 g; obtained in Example 7) according to the process of Example 3. The thus obtained compound showed the identical TLC and NMR values to those of the compound of Example 3.

### Example 9

### Synthesis of (±)-2-[N'-benzyl-N'-(2-hydroxyethyl)amino]-1-[3-(N-benzyl-N-methylsulfonylamino)phenyl]ethanol (Compound 3; Preparation Process 1)

The title compound was obtained from the compound (1.2 g; obtained in Example 8) according to the process of Example 4. The thus obtained compound showed the identical TLC and NMR values to those of the compound of Example 4.

### Example 10

### Synthesis (±)-2-[N'-benzyl-N'-[2-(9H-carbazol-2-yloxy)ethyl]amino]-1-[3-(N-benzyl-N-methylsulfonylamino)phenyl]ethanol (Compound 2; Preparation Process 1)

The title compound was obtained from the compound (1.1 g; obtained in Example 9) according to the process of Example 5. The thus obtained compound showed the identical TLC and NMR values to those of the compound of Example 5.

### Example 11

### Synthesis of (±)-2-[2-(9H-carbazol-2-yloxy)ethylamino]-1-[3-(methylsulfonylamino)phenyl]ethanol hydrochloride (Compound 1; Preparation Process 1)

The title compound was obtained from the compound (1 g; obtained in Example 10) according to the process of Example 6.

The thus obtained compound was shown to be identical to the title compound prepared according to the known method (JP-A-9-249623) by the fact that they had the same retention time in HPLC measurements.

### Example 12

### Synthesis of 2-chloro-1-[4-benzyloxy-3-(N-benzyl-N-methylsulfonylamino)phenyl]ethanone (Compound 6: Preparation Process 1)

### Step A. Synthesis of 4'-benzyloxy-3'-(N-benzyl-N-methylsulfonylamino)acetophenone

Potassium carbonate (26.2 g; mfd. by Wako Pure Chemical Industries), benzyl bromide (32.33 g; mfd. by Wako Pure Chemical Industries) and sodium iodide (5.16 g; mfd. by Wako Pure Chemical Industries) were added to a solution of 4'-benzyloxy-3'-(methylsulfonylamino)acetophenone (55 g; synthesized according to the process of Example 1) in dimethylformamide (140 mL) and the mixture was stirred at room temperature for 5 hours. The reaction solution was poured into water (250 mL) with stirring, washed off with ethyl acetate (501 mL) and stirred for 20 minutes. The crystal precipitated was separated by filtration and washed with heptane (215 mL). The crystal was transferred into another vessel, to which heptane (125 mL) was added, followed by stirring for 20 minutes. The crystal was separated by filtration, washed with heptane (125 mL) and dried under reduced pressure at 50°C to give the title compound (67.9 g) as a solid.
R_{f}=0.47 (1:1 ethyl acetate/n-hexane);
¹H-NMR (CDCl₃): 2.40 (3H, s), 2.87 (3H, s), 4.75 (2H, br.s), 5.18 (2H, s), 7.05 (1H, d, J=8.7), 7.20-7.25 (5H, m), 7.39-7.46 (5H, m), 7.61 (1H, d, J=2.1), 7.89 (1H, dd, J=8.7, 2.1).

### Step B. Synthesis of 2-chloro-1-[4-benzyloxy-3-(N-benzyl-N-methylsulfonylamino)phenyl]ethanone

A solution of sulfuryl chloride (0.35 mL; mfd. by Wako Pure Chemical Industries) in dichloromethane (6 mL) was added dropwise to a solution of the compound (1 g; obtained in the above step A) in dichloromethane (1.26 mL) and methanol (0.4 mL) at room temperature over 1 hour. After the reaction was completed, water (5 mL) was added and the reaction solution was separated. The organic layer was washed with a 0.1 N NaOH aqueous solution (5 mL × 3), dried and concentrated under reduced pressure. The residue was purified by silica gel chromatography (1:2 ethyl acetate/n-hexane) to give the title compound (1.05 g) as a light brown solid.
R_{f}=0.53 (1:1 ethyl acetate/n-hexane);
¹H-NMR (CDCl₃): 2.88 (3H, s), 4.46 (2H, s), 4.75 (2H, br.), 5.20 (2H, s), 7.09 (1H, d, J=8.8), 7.20-7.26 (5H, m), 7.41-7.46 (5H, m), 7.59 (1H, d, J=2.5), 7.92 (1H, dd, J=8.8, 2.5 ).

### Example 13

### Synthesis of (R)-2-chloro-1-[4-benzyloxy-3-(N-benzyl-N-methylsulfonylamino)phenyl]ethanol (Compound 5; Preparation Process 1)

In accordance with the process of Example 2, [(S,S)-N-(p-toluenesulfonyl)-1,2-diphenylethylenediamine](p-cymene) ruthenium complex (3.2 mg) was added to a solution of 2-chloro-1-[4-benzyloxy-3-(N-benzyl-N-methylsulfonylamino)phenyl]ethanone (222 mg; synthesized in Example 12) in a formic acid/triethylamine solution (0.5 mL) and tetrahydrofuran (1 mL). The mixture was stirred at room temperature for 8.5 hours.

After the reaction was completed, the reaction solution was added to ethyl acetate (5 mL) and water (5 mL), stirred vigorously, separated, washed with saturated brine, and then dried. After the solvent was distilled off under reduced pressure, the residue was purified by silica gel chromatography (1:3 ethyl acetate/hexane) and concentrated to give the title compound (222 mg).
R_{f}=0.42 (1:1 ethyl acetate/n-hexane);
¹H-NMR (CDCl₃): 2.83 (3H, s), 2.96 (1H, br.s), 3.35-3.45 (2H, m), 4.60-4.65 (1H ,m), 4.72 (2H, br.), 5.10 (2H,s), 6.97-7.00 (2H, m), 7.17-7.27 (6H, m), 7.37-7.43 (5H, m).
HPLC: Retention Time (R-form: 11.0 min (S-form: 13.2 min))
Column: CHIRALPAK AD (mfd. by DAICEL CHEMICAL INDUSTRIES; 4.6 mm ID × 250 mm);
Solvent: 1:1 hexane/ethanol;
Flow rate: 0.5 mL/min;
Detecting wave length: 254 nm;
Temperature: room temperature.

### Example 14

### Synthesis of (R)-1-[4-benzyloxy-3-(N-benzyl-N-methylsulfonylamino)phenyl]oxirane (Compound 4; Preparation Process 1)

The title compound was obtained from the compound (2 g; obtained in Example 13) according to the process of Example 3.
R_{f}=0.33 (1:2 ethyl acetate/n-hexane);,
¹H-NMR (CDCl₃): 2.61 (1H, dd, J=5.5, 2.5), 2.85 (3H, s), 3.03 (1H, dd, J=5.5, 4.0), 3.68 (1H, dd, J=4.0, 2.5), 4.73 (2H, br.), 5.12 (2H, s), 6.98 (1H, d, J=8.5), 7.02 (1H, d, J=2.2), 7.13 (1H, dd, J=8.5, 2.2), 7.20-7.25 (5H, m), 7.38-7.45 (5H, m).
HPLC: Retention Time (R-form: 16.2 min (S-form: 22.1 min))
Column: CHIRALPAK AD (mfd. by DAICEL CHEMICAL INDUSTRIES; 4.6 mm ID × 250 mm);
Solvent: 1:1 hexane/ethanol;
Flow rate: 0.5 mL/min;
Detecting wave length: 254 nm;
Temperature: 22°C.

### Example 15

### Synthesis of (±)-2-chloro-1-[4-benzyloxy-3-(N-benzyl-N-methylsulfonylamino)phenyl]ethanol (Compound 5; Preparation Process 1)

A solution of 1 M borane dimethyl sulfide in dichloromethane (2 mL; mfd. by Aldrich) was added to a solution of the compound (204 mg; obtained in Example 12) in anhydrous dichloromethane (5 mL) at room temperature and the mixture was stirred for 20 minutes. After the reaction was completed, 2 N HCl (5 mL) and methanol (1 mL) were added with ice cooling. Dichloromethane (10 mL) was added and the reaction solution was separated. The organic layer was dried and concentrated under reduced pressure. The residue was purified by silica gel chromatography (1:2 ethyl acetate/n-hexane) to give the title compound (137 mg) as a colorless solid.

### Example 16

### Synthesis of (±)-1-[4-benzyloxy-3-(N-benzyl-N-methylsulfonylamino)phenyl]oxirane (Compound 4; Preparation Process 1)

The title compound was obtained from the compound (1.85 g; obtained in Example 15) according to the process of Example 3. The thus obtained compound showed the identical TLC and NMR values to those of the compound of Example 14.

### Example 17

### Synthesis of (R)-2-[N'-benzyl-N'-(2-hydroxyethyl)amino]-1-[4-benzyloxy-3-(N-benzyl-N-methylsulfonylamino)phenyl]ethanol (Compound 3; Preparation Process 1)

N-benzylethanolamine (3.6 mL; mfd. by TOKYO KASEI KOGYO) was added to the compound (6.83 g; obtained according to the process of Example 14) and the mixture was stirred at 100°C for 24 hours. After cooled down, the mixture was purified by silica gel chromatography (1:2 to 1:1 ethyl acetate/n-hexane) and concentrated under reduced pressure to give a 3.3:1 mixture of the title compound and its isomer (5.78 g) as a yellowish white amorphous solid.
R_{f}=0.51 (1:9 methanol/chloroform);
¹H-NMR (CDCl₃): 2.45-2.64 (2H, m), 2.70-2.81 (1H, m), 2.85 (2.3H, s), 2.89 (0.7H, s), 3.46-3.64 (4H, m), 3.71-3.83 (1.2H, m), 4.45-4.51 (0.8H, m), 4.74 (2H, br.), 5.10 (1.5H, s), 5.15 (0.5H, s), 6.94-7.06 (2H, m), 7.18-7.48 (16H, m).

### Example 18

### Synthesis of (R)-2-[N'-benzyl-N'-[2-(9H-carbazol-2-yloxy)ethyl]amino]-1-[4-benzyloxy-3-(N-benzyl-N-methylsulfonylamino)phenyl]ethano] (Compound 2; Preparation Process 1)

Carbon tetrabromide (10.99 g; mfd. by Wako Pure Chemical Industries) was added to a solution of the compound (12.39 g; obtained in Example 17) and triphenylphosphine (6.96 g; mfd. by Wako Pure Chemical Industries) in anhydrous dichloromethane (221 mL) at -18°C and the mixture was stirred for 30 minutes (the intermediate brominated compound: R_{f}=0.58 (1:1 ethyl acetate/n-hexane)). Further, tetrahydrofuran (221 mL), 2-hydroxycarbazole (4.05 g; mfd. by Aldrich) and 2 N NaOH (22.1 mL) were added and the mixture was stirred at room temperature for 20 hours. The solvent was distilled off under reduced pressure from the reaction solution. The residue was dissolved in ethyl acetate (170 mL) and washed with 2 N NaOH (50 mL × 3) and then saturated brine. The organic layer was dried and the solvent was distilled off under reduced pressure. The residue was purified by silica gel column chromatography (1:10 to 1:2 ethyl acetate/n-hexane) to give the title compound (11.75 g) as a light yellow amorphous solid.
R_{f}=0.30 (1:1 ethyl acetate/n-hexane);
¹H-NMR (DMSO-d₆): 2.50-2.53 (2H, m), 2.81-2.89 (2H, m), 2.94 (3H, s), 3.72 (2H, br.s), 3.96 (2H,m), 4.50-4.58 (1H, m), 4.70 (2H, br.s), 4.96 (1H, d, J=3.9), 5.10 (2H, s), 6.71 (1H, dd, J=8.4, 2.2), 6.90 (1H, d, J=2.2), 7.01 (1H, d, J=8.4), 7.04 (1H, d, J=2.4), 7.07-7.48 (19H, m), 7.94 (1H, d, J=8.4), 7.96 (1H, d), 11.06 (1H, s).

### Example 19

### Synthesis of (R)-2-[N-[2-(9H-carbazol-2-yloxy)ethyl]amino]-1-[4-hydroxy-3-(methylsulfonylamino)phenyl]ethanol hydrochloride (Compound 1; Preparation Process 1)

10% Palladium/carbon (413 mg; mfd. by Merck) was added to a solution of the compound (2 g; obtained in Example 18) in tetrahydrofuran (45 mL) and methanol (45 mL). The mixture was stirred under a hydrogen atmosphere at room temperature for 16 hours. After the reaction was completed, the reaction solution was filtered, washed with 1:1 tetrahydrofuran/methanol (50 mL). To the filtrate were added sequentially ethyl acetate (350 mL) and a 0.1 N HCl/ethanol solution (30mL; mfd. by TOKYO KASEI KOGYO), followed by stirring. The crystal precipitated was separated by filtration and dried under reduced pressure at 55°C to give the title compound (992 mg) as a white crystal. R_{f}=0.31 (1:5 10% aqueous ammonia-containing methanol/chloroform).

The thus obtained compound was shown to be identical to the title compound prepared according to the known method (JP-A-9-249623) by the fact that they had the same retention time in HPLC measurements.

### Example 20

### Synthesis of 2-chloro-1-[4-chloro-3-(N-benzyl-N-methylsulfonylamino)phenyl]ethanone (Compound 6; Preparation Process 1)

### Step A. Synthesis of 4'-chloro-3'-(N-benzyl-N-methylsulfonylamino)acetophenone

Potassium carbonate (31.7 g; mfd. by KANTO CHEMICAL) and benzyl bromide (21.6 g; mfd. by Wako Pure Chemical Industries) were added to a solution of 4'-chloro-3'-(methylsulfonylamino)acetophenone (28.43 g; prepared according to the process of Example 1) in dimethylformamide (50 mL) and the mixture was stirred at room temperature for 24 hours. Water (200 mL), ethyl acetate (200 mL) and heptane (50 mL) were added and the mixture was stirred vigorously. The reaction solution was separated and the aqueous layer was extracted with ethyl acetate. The combined organic layers were washed with water and saturated brine, and the solvent was distilled off under reduced pressure. The residue was purified by silica gel column chromatography (1:4 to 3:7 ethyl acetate/n-hexane) to give the title compound (27.63 g) as a light yellow oil.
R_{f}=0.44 (1:1 ethyl acetate/n-hexane);
¹H-NMR (CDCl₃): 2.40 (3H, s), 3.08 (3H, s), 4.58 (1H, br.), 5.09 (1H, br.), 7.20-7.30 (5H, m), 7.53 (1H, d, J=8.2), 7.56 (1H, d, J=2.2), 7.82 (1H, dd, J=8.2, 2.2).

### Step B.Synthesis of 2-chloro-1-[4-chloro-3-(N-benzyl-N-methylsulfonylamino)phenyl]ethanone

A solution of sulfuryl chloride (0.43mL; mfd. by Wako Pure Chemical Industries) in dichloromethane (0.65mL) was added dropwise to a solution of the compound (1 g; obtained in the above Step A) in dichloromethane (1.54 mL) and methanol (0.5 mL) at room temperature over 1 hour. After the reaction was completed, water (5 mL) was added and the reaction solution was separated. The organic layer was washed with a 0.1 N NaOH aqueous solution (10 mL × 3), dried and concentrated under reduced pressure to give the title compound (1.19 g).
R_{f}=0.63 (1:1 ethyl acetate/n-hexane);
¹H-NMR (CDCl₃): 3.09 (3H, s), 4.44 (2H, s), 4.55 (1H, br.), 5.15 (1H, br.), 7.20-7.30 (5H, m), 7.55 (1H, d, J=2.2), 7.58 (1H, d, J=8.2), 7.85 (1H, dd, J=8.2, 2.2).

### Example 21

### Synthesis of (R)-2-chloro-1-[4-chloro-3-(N-benzy]-N-methylsulfonylamino)phenyl]ethanol (Compound 5; Preparation Process 1)

In accordance with the process of Example 2, [(S,S)-N-(p-toluenesulfonyl)-1,2-diphenylethylenediamine](p-cymene) ruthenium complex (3.9 mg) was added to a solution of 2-chloro-1-[4-chloro-3-(methylsulfonylamino)phenyl]ethanone (227 mg; obtained in Example 20) in formic acid/triethylamine solution (0.5 mL) and tetrahydrofuran (0.5 mL). The mixture was stirred at room temperature for 40 minutes.

After the reaction was completed, the reaction solution was added to ethyl acetate (5 mL) and water (5 mL), stirred vigorously, separated, and then washed with saturated brine. The organic layer was dried and the solvent was distilled off under reduced pressure. The residue was purified by silica gel chromatography (1:3 ethyl acetate/n-hexane) and concentrated to give the title compound (181 mg).
R_{f}=0.48 (1:1 ethyl acetate/n-hexane);
¹H-NMR(CDCl₃): 2.89 (1H, m), 3.05 (3H, s), 3.32-3.50 (2H, m), 4.52 (1H, br.), 4.66-4.71 (1H, m), 5.07 (1H, br.), 6.95-7.04 (1H, m), 7.17-7.27 (6H, m), 7.43 (1H, d, J=8.2).
HPLC: Retention Time (R-form: 20.6 min (S-form: 18.4 min))
Column: CHIRALPAK AS (mfd. by DAICEL CHEMICAL INDUSTRIES; 4.6 mm ID × 250 mm);
Solvent: 85:15 n-hexane/ethanol;
Flow rate: 0.5 mL/min;
Detecting wave length: 254 nm;
Temperature: 40°C.

### Example 22

### Synthesis of (±)-2-chloro-1-[4-chloro-3-(N-benzyl-N-methylsulfonylamino)phenyl]ethanol (Compound 5; Preparation Process 1)

A solution of 1 M borane dimethyl sulfide in dichloromethane (1 mL; mfd. by Aldrich) was added to a solution of the compound (181 mg; obtained in Example 20) in anhydrous dichloromethane (5 mL) at room temperature and the mixture was stirred for 20 minutes. After the reaction was completed, the reaction solution was stirred with water and separated. The organic layer was dried and concentrated under reduced pressure. The residue was purified by silica gel chromatography (1:2 ethyl acetate/n-hexane) to give the title compound (109 mg).

### Example 23

### Synthesis of (R)-2-[2-(9H-carbazol-2-yloxy)ethylamino]-1-[3-(methylsulfonylamino)phenyl]ethanol (Compound 1; Preparation Process 2)

A mixture of the compound (9.58 g; obtained in Example 3) and the intermediate (10 g) in 2-propanol (70 mL) was stirred with heating at the internal temperature of 80°C for 29.5 hours. After the reaction mixture was cooled down, the solvent was distilled off under reduced pressure to give the dibenzyl compound (21 g) as a light yellow amorphous solid. The thus obtained compound showed the identical TLC and R_{f} values to those of the compound of Example 5.

Next, 10% palladium/carbon (627 mg; mfd. by Merck) was added to a solution of the thus obtained dibenzyl compound (21 g) in ethanol (1075 mL). The mixture was stirred under a hydrogen atmosphere at the internal temperature of 80°C for 4 hours. After the reaction mixture was cooled down, tetrahydrofuran (516 mL) was added. The mixture was filtered and the filtrate was concentrated under reduced pressure to give the title compound (13.12 g).
R_{f}=0.13 (1:9 methanol/chloroform).

The thus obtained compound was shown to be identical to the title compound prepared according to the known method (JP-A-9-249623) by the fact that they had the same retention time in HPLC measurements.

### Example 24

### Synthesis of (R)-[5-[(2-iodo-1-triethylsilyloxy)ethyl]-2-benzyloxyphenyl](methylsulfonyl)benzylamine (Compound 10; Preparation Process 3)

Sodium iodide (48 g; mfd. by Wako Pure Chemical Industries) was added to a solution of the compound (4.46 g; obtained in Example 13) in acetone (120 mL). The mixture was heated to reflux for 72 hours and then cooled down. After insoluble substance was filtered off, the filtrate was extracted with ethyl acetate and washed with water and then saturated brine. The organic layer was dried and the solvent was distilled off under reduced pressure to give a iodinated compound (4.95 g).
R_{f}=0.46 (1:1 ethyl acetate/n-hexane);
¹H-NMR (CDCl₃): 2.57 (1H, br.), 2.85 (3H, s), 3.15 (1H, dd, J=10.2, 8.2), 3.23 (1H, dd, J=10.2, 4.1), 4.58 (1H, dd, J=8.2, 4.1), 4.73 (2H, br.), 5.12 (2H, s), 6.98 (1H, s), 7.00 (1H, d, J=8.6), 7.16-7.29 (6H, m), 7.37-7.45 (5H, m).

To a solution of the thus obtained iodinated compound (4.8 g) in dimethylformamide (20 mL) were added imidazole (1.61 g; mfd. by TOKYO KASEI KOGYO) and dimethylaminopyridine (92 mg; mfd. by TOKYO KASEI KOGYO), followed by addition of triethylsilane chloride (1.94 g; mfd. by Shin-Etsu Chemical) at room temperature. The mixture was stirred 40 minutes, diluted with ethyl acetate (60 mL) and heptane (20 mL), washed sequentially with water (30 mL), 2% copper sulfate solution (30 mL), water (30 mL) and saturated brine (30 mL). The organic layer was dried, and the solvent was distilled off under reduced pressure. The residue was purified by column chromatography (1:10 ethyl acetate/n-hexane) to give the title compound (5.1 g), which was then recrystallized from ethyl acetate/n-hexane.
m.p. 110 to 111°C;
R_{f}=0.77 (1:2 ethyl acetate/n-hexane);
¹H-NMR (CDCl₃): 0.40-0.50 (6H, m), 0.83 (9H, t, J=7.7), 2.86 (3H,s), 3.11-3.16 (2H, m), 4.53-4.59 (1H, m), 4.74 (2H, br.), 5.12 (2H, s), 6.98 (1H, d, J=8.5), 6.98 (1H, d, J=2.2), 7.18-7.24 (6H, m), 7.38-7.45 (5H, m).

### Example 25

### Synthesis of (±)-[5-[(2-iodo-1-triethylsilyloxy)ethyl]-2-benzyloxyphenyl](methylsulfonyl)benzylamine (Compound 10; Preparation Process 3)

The title compound (9.86 g) was obtained according to the process of Example 24, with the exception that the compound of Example 15 (10.47 g) was used.

### Example 26

### Synthesis of (R)-[5-[2-[N-benzyl-N-(2-hydroxyethyl)amino]-1-triethylsilyloxy)ethyl]-2 benzyloxyphenyl](methylsulfonyl)benzylamine (Compound 10; Preparation Process 3)

N-benzylethanolamine (5.9 mL; mfd. by TOKYO KASEI KOGYO) was added to the compound (10 g; obtained in Example 24) and the mixture was stirred with heating at 100°C for 15 hours. After cooling down, the mixture was purified by silica gel chromatography (1:1 ethyl acetate/n-hexane) and concentrated under reduced pressure to give the title compound (8.8 g) as a yellowish white amorphous solid.
R_{f}=0.69 (1:1 ethyl acetate/n-hexane);
¹H-NMR (CDCl₃): 0.28-0.38 (6H, m), 0.76 (9H, t, J=7.7), 2.43-2.60 (3H, m), 2.68 (1H, dd, J=13.4, 6.3), 2.86 (3H, s), 3.35 (2H, m), 3.54-3.65 (2H, m), 4.30-4.37 (1H, m), 4.6-4.9 (2H, m), 5.12 (2H, s), 6.95 (1H, d, J=8.5), 7.12-7.28 (12H, m), 7.38-7.46 (5H, m).

### Example 27

### Synthesis of (R)-[5-[2-[N-benzyl-N-[2-(9H-carbazol-2-yloxy)]ethyl]amino]-1-triethylsilyloxy)ethyl]-2-benzyloxyphenyl](methylsulfonyl)benzylamine (Compound 10; Preparation Process 3)

Carbon tetrabromide (6.72 g; mfd. by Wako Pure Chemical Industries) was added to a solution of the compound (8.8 g; obtained in Example 26) and triphenylphosphine (4.25 g; mfd. by Wako Pure Chemical Industries) in anhydrous dichloromethane (70 mL) at -20°C, and the mixture was stirred for 20 minutes (the intermediate brominated compound: R_{f}=0.80 (1:1 ethyl acetate/n-hexane)). Further, tetrahydrofuran (70 mL), 2-hydroxycarbazole (2.47 g; mfd. by Aldrich) and 2 N NaOH (13.5 mL) were added and the mixture was stirred at room temperature for 1 hour. The solvent was distilled off under reduced pressure from the reaction solution. The residue was dissolved in toluene (130 mL) and washed with 2 N NaOH (20 mL × 3) and saturated brine. The organic layer was dried and the solvent was distilled off under reduced pressure. The residue was purified by silica gel column chromatography (1:10 to 1:3 ethyl acetate/n-hexane) to give the title compound (7.42 g) as a light yellow amorphous solid.
R_{f}=0.30 (1:4 ethyl acetate/n-hexane);
¹H-NMR (CDCl₃): 0.26-0.42 (6H, m), 0.76 (9H, t, J=7.7), 2.67-2.90 (4H, m), 2.86 (3H, s), 3.65-3.84 (4H, m), 4.46 (1H, m), 4.60-4.86 (2H, m), 4.94 (2H, s), 6.73 (1H, dd, J=8.4, 2.2), 6.80 (1H, d, J=8.7), 6.84 (1H, br.s), 7.04-7.42 (20H, m), 7.87 (1H, d, J=8.4), 7.95 (1H, d, J=7.5), 8.37 (1H, br.s).

### Example 28

### Synthesis of (R)-2-[N-[2-(9H-carbazol-2-yloxy)]ethyl]amino-1-(4-hydroxy-3-methylsulfonylamino)phenyl-1-ethanol hydrochloride (Compound 1; Preparation Process 3)

Acetic acid (2.17 mL) and tetrabutylammonium fluoride (21.7 mL; mfd. by Aldrich) were sequentially added to a solution of the compound (7.42 g; obtained in Example 27) in anhydrous tetrahydrofuran (109 mL) and the mixture was stirred at room temperature for 6 hours. The mixture was diluted with ethyl acetate (120 mL) and washed with an aqueous sodium bicarbonate solution (12.5 g/120 mL) and saturated brine. The organic layer was dried and the solvent was distilled off under reduced pressure to give the title compound (6.54 g) as a yellow amorphous solid. R_{f}=0.68 (1:1 ethyl acetate/n-hexane). The thus obtained compound showed the identical TLC and NMR values to those of the compound of Example 18.

20% Palladium hydroxide/carbon (571 mg; mfd. by N.E. CHEMCAT) was added to a solution of the thus obtained compound (450 mg) in tetrahydrofuran (20 mL) and methanol (20 mL). The mixture was stirred under a hydrogen atmosphere at room temperature for 3 hours. After the reaction was completed, the mixture was filtered and washed with 1:1 tetrahydrofuran/methanol (9 mL). To the filtrate, 0.1 N HCl/ethanol solution (6.8 mL; mfd. by TOKYO KASEI KOGYO) was added, followed by stirring. The crystal precipitated was separated by filtration and dried under reduced pressure at 50°C to give the title compound (155 mg) at a white solid.

The thus obtained compound was shown to be identical to the title compound prepared according to the known method (JP-A-9-249623) by the fact that they had the same retention time in HPLC measurements.

### Example 29

### Synthesis of (R)-[3-[(2-iodo-1-triethylsilyloxy)ethyl]phenyl](methylsulfonyl)benzylamine (Compound 10; Preparation Process 3)

Sodium iodide (48 g; mfd. by Wako Pure Chemical Industries) was added to a solution of the compound (3.39 g; obtained in Example 2) in acetone (120 mL) and the mixture was heated to reflux for 72 hours. After the mixture was cooled down, insoluble substance was filtered and the filtrate was extracted with ethyl acetate and washed with water and saturated brine. The organic layer was dried and the solvent was distilled off under reduced pressure to give the iodinated compound (4.05 g).
R_{f}=0.45 (1:1 ethyl acetate/n-hexane);
¹H-NMR (CDCl₃): 2.59 (1H, d), 2.95 (3H, s), 3.24-3.30 (1H, m), 3.38-3.42 (1H, m), 4.71-4.76 (1H, m), 4.85 (2H, s), 7.19-7.35 (9H, m).

Imidazole (2.04 g; mfd. by TOKYO KASEI KOGYO) and dimethylaminopyridine (11 mg; mfd. by TOKYO KASEI KOGYO) were added to a solution of the above obtained iodinated compound (4 g) in dimethylformamide (100 mL). Triethylsilane chloride (1.8 g; mfd. by Shin-Etsu Chemical) was added at room temperature and the mixture was stirred for 40 minutes. The mixture was diluted with ethyl acetate and washed sequentially with water, 2% copper sulfate solution, water and saturated brine, and then dried. The solvent was distilled off under reduced pressure and the residue was purified by column chromatography (1:9 ethyl acetate/n-hexane) to give the title compound (4.45 g).
R_{f}=0.84 (1:1 ethyl acetate/n-hexane);
¹H-NMR (CDCl₃): 0.46 (6H, m), 0.86 (9H, t, J=7.7), 2.95 (3H, s), 3.24 (2H, d, J=5.8), 4.67 (1H, t, J=5.8), 4.80-4.91 (2H, m), 7.18-7.31 (9H, m).

### Example 30

### Synthesis of (R)-2-[N-[2-(9H-carbazol-2-yloxy)]ethyl]amino-1-(4-hydroxy-3-methylsulfonylamino)phenyl-1-ethanol (Compound 1: Preparation Process 4)

A solution of the compound (163 mg; obtained in Example 24) and the intermediate (395 mg) in anhydrous dimethylacetamide (0.5 mL) was stirred at 70°C for 24 hours. The mixture was diluted with ethyl acetate to the volume of about 12 mL. The crystal precipitated was separated by filtration (intermediate) and the filtrate was concentrated under reduced pressure. The residue was purified by silica gel chromatography (first: chloroform; second: 1:5 ethyl acetate/n-hexane) to give (R)-[5-[2-[N-benzyl-N-[2-(9H-carbazol-2-yloxy)]ethyl]amino]-1-triethylsilyloxy)ethyl]-2-benzyloxyphenyl](methylsulfonyl)benzylamine (163 mg). The thus obtained compound showed the identical TLC and NMR values to those of the compound of Example 27.

Next, the compound obtained above was deprotected according to the process of Example 28 to give the title compound.

The thus obtained compound was shown to be identical to the title compound prepared according to the known method (JP-A-9-249623) by the fact that they had the same retention time in HPLC measurements.

### Example 31

### Synthesis of (R)-2-[N-[2-(6-hydroxy-9H-carbazol-2-yloxy)]ethyl]amino-1-(3-methylsulfonylamino)phenylethanol hydrochloride (Preparation Process 1)

### Step A. Synthesis of 2-methoxy-6-hydroxycarbazole

Water (30 mL) and concentrated hydrochloric acid (160 mL) were added to 2-nitro-4-methoxyaniline (16.8 g), and the mixture was stirred at room temperature for 20 minutes and then at 70°C for 75 minutes. After cool down, an aqueous solution (30 mL) containing sodium nitrite (11.5 g) was added dropwise with ice cooling such that the temperature of the reaction solution did not exceed 5°C. After the addition, the mixture was stirred for 1 hour while the temperature was maintained at 10°C. The reaction solution was filtered and the residue was washed with water (50 mL). The filtrate was cooled with ice. An aqueous solution (120 mL) in which sodium hydrogencarbonate (123 g) and 1,4-benzoquinone (12.3 g) had been mixed was added dropwise over 1 hour. After the addition was completed, the reaction solution was stirred with ice cooling for 4 hours and then filtered. The crystal was washed with water and dried. The thus obtained crystal was dissolved in methanol (200 mL) and acetic acid (20 mL). 10% Palladium/carbon (1.0 g) was added and the mixture was stirred under a hydrogen atmosphere at room temperature for 3 hours. The reaction solution was filtered and the residue was washed with methanol (30 mL). To the filtrate, concentrated aqueous ammonia (50 mL) was added dropwise with ice cooling over 5 minutes. The reaction solution was allowed to warm to room temperature, and then stirred for 12 hours. The reaction solution was filtered. The crystal was washed with water and dried in vacuo. The thus obtained crude product was purified by silica gel column chromatography (3:1 to 0:1 hexane/ethyl acetate) to give the title compound (2.71 g).
R_{f}=0.38 (1:1 ethyl acetate/n-hexane);
¹H-NMR (DMSO-d₆): 3.82 (3H, s), 6.68 (1H, dd, J=2.2, 8.5), 6.77 (1H, dd, J=2.2, 8.5), 6.88 (1H, d, J=2.2), 7.20 (1H, d, J=8.5), 7.30 (1H, d, J=2.2), 7.83 (1H, d, J=8.5), 8.82 (1H, br), 10.73 (1H, br).

### Step B.Synthesis of 2-methoxy-6-benzyloxycarbazole

The compound (3.90 g; synthesized in the Step A) was dissolved in acetone (90 mL) and DMF (6 mL), to which potassium carbonate (10.1 g) and benzyl bromide (3.12 g) were added. The mixture was stirred at room temperature for 25 hours. Benzyl bromide (1.56 g) was added and the mixture was further stirred for 24 hours. Water (500 mL) was added to the reaction solution and the crystal precipitated was separated by filtration, washed with water and then dried in vacuo. The thus obtained crude product was added to ethyl acetate (40 mL) and the mixture was stirred for 10 minutes. The crystal was separated by filtration and dried in vacuo to give the title compound (3.28 g).
R_{f}=0.66 (1:1 ethyl acetate/n-hexane);
¹H-NMR (DMSO-d₆): 3.83 (3H, s), 5.16 (2H, s), 6.73 (1H, dd, J=2.2, 8.5), 6.92 (1H, d, J=2.2), 6.99 (1H, dd, J=2.5, 8.5), 7.30-7.43 (4H, m), 7.50-7.52 (2H, m), 7.67 (1H, d, J=2.2), 7.92 (1H, d, J=8.5), 10.90 (1H, br.).

### Step C.Synthesis of 2-hydroxy-6-benzyloxycarbazole

The compound (5.93 g; obtained in the Step B) was dissolved in DMSO (110 mL). Sodium cyanide (5.75 g) was added to the mixture and was stirred at 170°C for 7 hours. Water (150 mL) was added to the reaction solution, which was then extracted with ethyl acetate. The organic layer was washed with water and dried, and the solvent was distilled off under reduced pressure. The residue was purified by silica gel column chromatography (1:1 hexane/ethyl acetate) to give a 1:1 mixture (1.24 g) of the title compound and 2-methoxy-6-hydroxycarbazole.
R_{f}=0.69 (1:1 ethyl acetate/n-hexane).
The following is a spectral data of 2-hydroxy-6-benzyloxycarbazole.
¹H-NMR (DMSO-d₆): 5.15 (2H, s), 6.59 (1H, dd, J=2.2, 8.2), 6.76 (1H, d, J=2.5), 6.95 (1H, dd, J=2.5, 8.5), 7.26 (1H, d, J=8.5), 7.32-7.43 (3H, m), 7.49-7.52 (2H, m), 7.60 (1H, d, J=2.5), 7.80 (1H, d, J=8.2), 9.35 (1H, br), 10.72 (1H, br).

### Step D. Synthesis of (R)-2-[N-benzyl-N-[2-(6-benzyloxy-9H-carbazol-2-yloxy)]ethyl]amino-1-[3-(N-benzyl-N-methylsulfonylamino)]phenylethanol

N-Bromosuccinimide (2.04 g; mfd. by TOKYO KASEI KOGYO) was added to a solution of the compound (5.3 g; obtained in Example 9) and triphenylphosphine (2.98 g; mfd. by Wako Pure Chemical Industries) in anhydrous dichloromethane (100 mL) at -15°C and the mixture was stirred for 10 minutes (the brominated compound: R_{f}=0.91 (1:9 methanol/chloroform)). The reaction was completed, the mixture was purified by silica gel column (4:1 to 2:1 n-hexane/ethyl acetate) and concentrated.

The 1:1 mixture of 2-hydroxy-6-benzyloxycarbazole and 2-methoxy-6-hydroxycarbazole (1.0 g; obtained in the Step C) was dissolved in tetrahydrofuran (25 mL), to which 2 N sodium hydroxide aqueous solution (3.45 mL) was added at room temperature. A previously prepared solution of the above brominated compound in tetrahydrofuran (25 mL) was added all at once and the mixture was stirred at room temperature for 17 hours. The solvent was distilled off under reduced pressure. The residue was dissolved in ethyl acetate, washed sequentially with 2 N sodium hydroxide aqueous solution and water and then dried. The solvent was distilled off. The residue was purified by silica gel column chromatography (3:1 to 1:1 hexane/ethyl acetate) to give a 1:1 mixture (2.71 g) of the title compound and a by-product ((R)-2-[N-benzyl-N-[2-(2-methoxy-9H-carbazol-6-yloxy)]ethyl]amino-1[3-(N-benzyl-N-methylsulfonylamino)]phenylethanol).

### Step E. Synthesis of (R)-2-[N-[2-(6-hydroxy-9H-carbazol-2-yloxy)]ethyl]amino-1-(3-methylsulfonylamino)phenylethanol hydrochloride

The mixture (2.4 g) of (R)-2-[N-benzyl-N-[2-(6-benzyloxy-9H-carbazol-2-yloxy)]ethyl]amino-1-[3-(N-benzyl-N-methylsulfonylamino)]phenylethanol and the abovementioned by-product was dissolved in a mixed solvent of tetrahydrofuran (35 mL) and methanol (35 mL), to which acetic acid (2.4 mL) was added. 20% Palladium hydroxide/carbon (1.2 g) was added under an argon atmosphere. After the atmosphere was replaced with hydrogen, the mixture was stirred for 15 hours. The catalyst was filtered and washed, and the filtrate was evaporated under reduced pressure. The residue was purified by silica gel column chromatography (19:1 to 8:1 chloroform/methanol). An alcoholic 0.5 N hydrochloric acid (3.9 mL) was added to the fraction containing the title compound as free form. The resulting mixture was concentrated. The crystal precipitated was separated by filtration, washed with cooled methanol and then dried to give the title compound (370 mg).
¹H-NMR (DMSO-d₆): 3.00 (3H, s), 3.05-3.53 (4H, m), 4.33-4.42 (2H, m), 5.02 (1H, d, J=9.9), 6.27 (1H, br), 6.75 (1H, dd, J=2.2, 8.5), 6.80 (1H, dd, J=2.2, 8.5), 6.95 (1H, d, J=2.2), 7.13-7.24 (3H, m), 7.31-7.39 (3H, m), 7.88 (1H, d, J=8.5), 8.88 (1H, br), 8.99 (1H, br), 9.24 (1H, br), 9.86 (1H, br), 10.85 (1H, br).

All the publications, patents and patent applications cited herein are incorporated herein by reference in their entireties.

### INDUSTRIAL APPLICABILITY

The present invention provides novel processes for the preparation of tricyclic amino alcohol derivatives or salts thereof which are useful for treating and preventing diabetes, obesity, hyperlipidemia and the like, and intermediates useful for the processes.

## Claims

1. A process for the preparation of a compound of the formula (1): 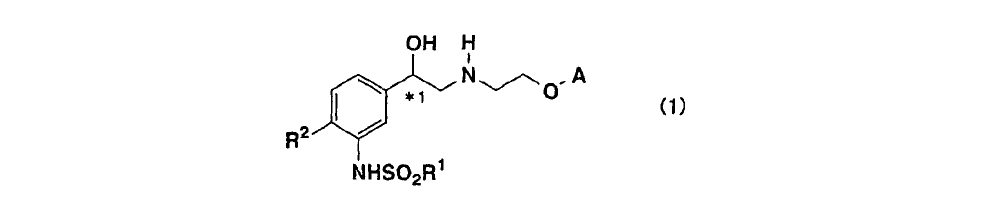 wherein R¹ represents a lower alkyl group or a benzyl group; R² represents a hydrogen atom, a halogen atom or a hydroxyl group; *1 represents an asymmetric carbon atom; and A represents one of the following groups: 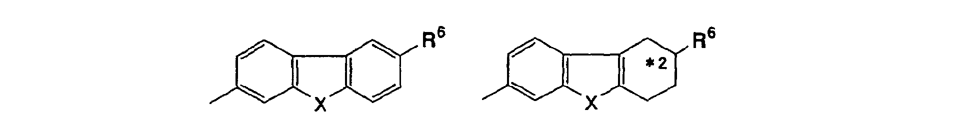 wherein X represents NH, O or S; R⁶ represents a hydrogen atom, a hydroxyl group, an amino group or an acetylamino group; and *2 represents an asymmetric carbon atom when R⁶ is not a hydrogen atom,
said process comprising:
chlorinating a compound of the formula (7): 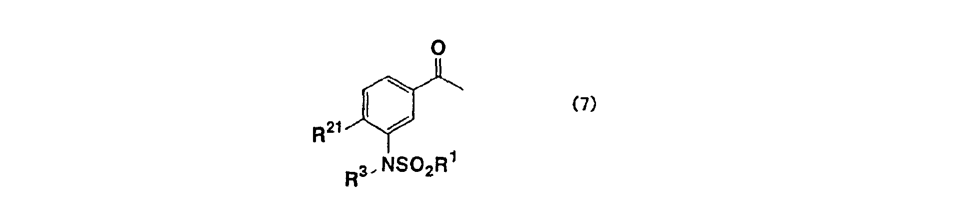 wherein R¹ is as defined above; R²¹ represents a hydrogen atom, a halogen atom or a protected hydroxyl group; and R³ represents an amino-protecting group, to give a compound of the formula (6): 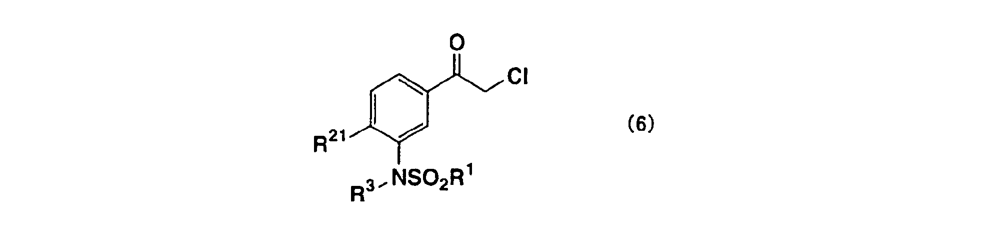 wherein R¹, R²¹ and R³ are as defined above; and then,
reducing the compound of the formula (6) to give a chlorohydrin compound of the formula (5): 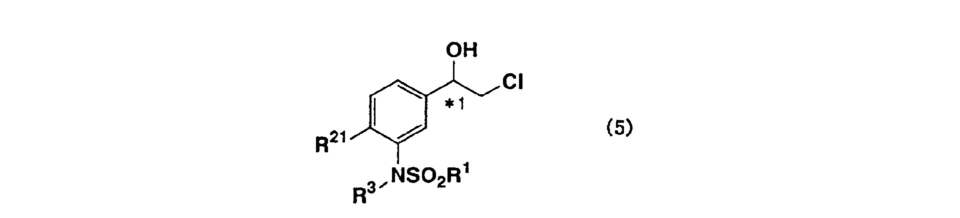 wherein R¹, R²¹, R³ and *1 are as defined above;
and then,
(i) treating the chlorohydrin compound of the formula (5) with an alkali to give an epoxy compound of the formula (4): 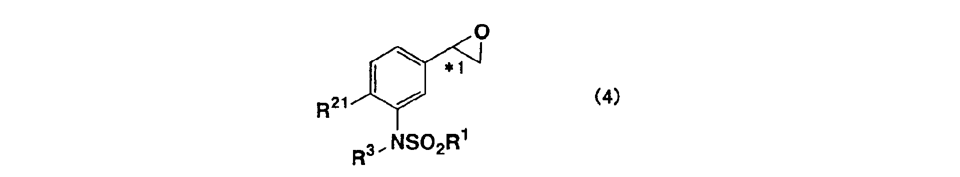 wherein R¹, R²¹, R³ and *1 are as defined above; and then,
(i-a) reacting the epoxy compound of the formula (4) with a compound of the formula (8): 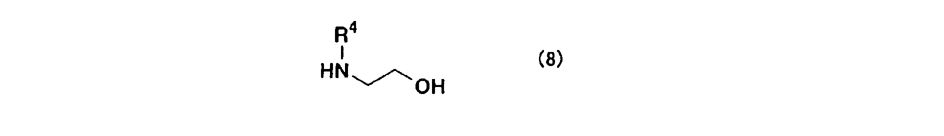 wherein R⁴ represents an amino-protecting group, to give a dialcohol compound of the formula (3): 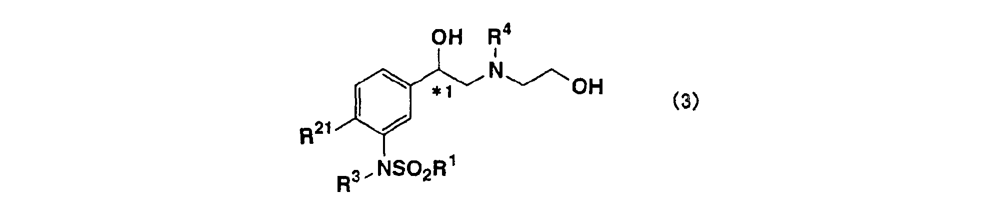 wherein R¹, R²¹, R³, R⁴ and *1 are as defined above;
brominating the primary alcohol, and then reacting the brominated product with a compound represented by A'-OH wherein A' represents one of the following groups: 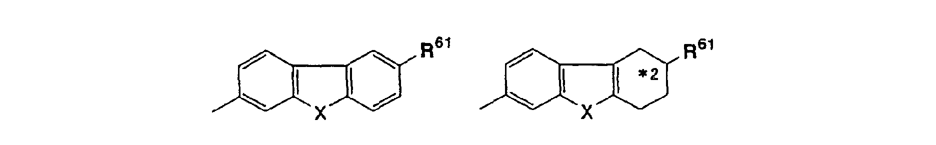 wherein X represents NH, O or S; R⁶¹ represents a hydrogen atom, a protected hydroxyl group, a protected amino group or an acetylamino group; and *2 represents an asymmetric carbon atom when R⁶¹ is not a hydrogen atom, to give an amino alcohol compound of the formula (2): 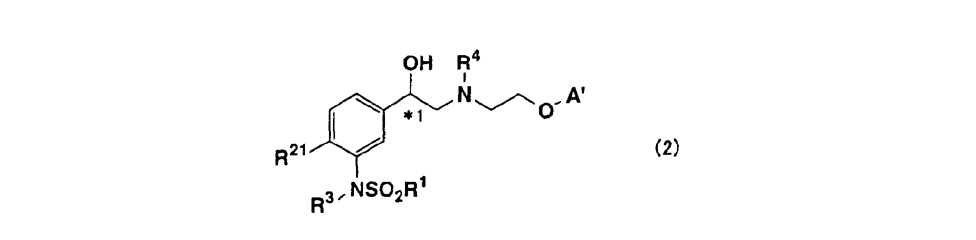 wherein R¹, R²¹, R³, R⁴, A' and *1 are as defined above; or
(i-b) reacting the compound of the formula (4) with a compound of the formula (9): 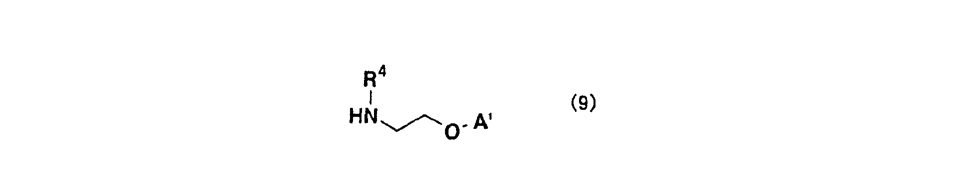 wherein R⁴ and A' are as defined above, to give a compound of the formula (2);
or, alternatively,
(ii) protecting the hydroxyl group of the compound of the formula (5) with a protecting group R⁵, or halogenating the compound of the formula (5) followed by protecting the hydroxyl group with a protecting group R⁵ to give a compound of the formula (10): 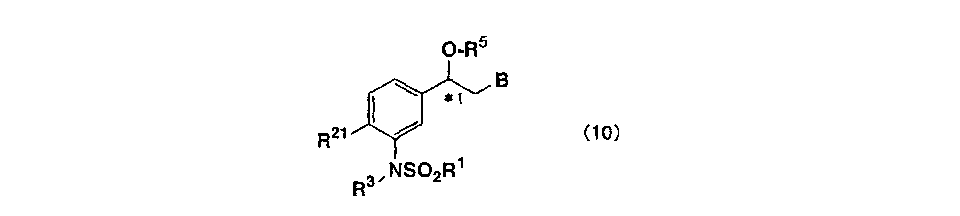 wherein R⁵ represents a hydroxyl-protecting group; B represents a halogen atom; and R¹, R²¹, R³ and *1 are as defined above; and then,
(ii-a) reacting the compound of the formula (10) with the compound of the formula (8) to give an alcohol compound of the formula (3a): 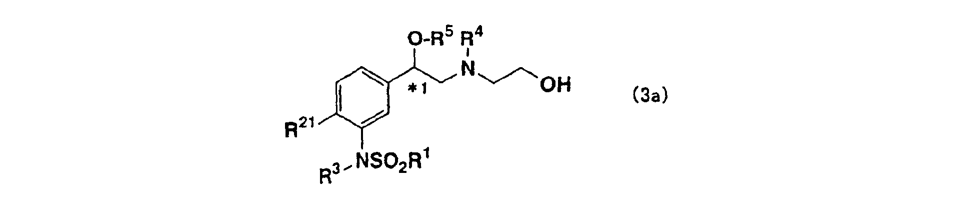 wherein R¹, R²¹, R³, R⁴, R⁵ and *1 are as defined above;
brominating the alcohol, and then reacting the brominated product with a compound represented by the A'-OH set forth above to give an amino alcohol compound of the formula (2a): 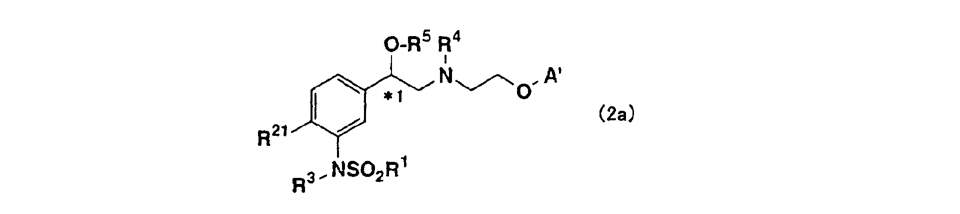 wherein R¹, R²¹, R³, R⁴, R⁵, A' and *1 are as defined above; or
(ii-b) reacting the compound of the formula (10) with the compound of the formula (9) to give an amino alcohol compound of the formula (2a);
and then,
simultaneously or sequentially removing the protecting groups of the thus obtained compound of the formula (2) or (2a) to give a compound of the formula (1).

2. A process for the preparation of a compound of the formula (1): 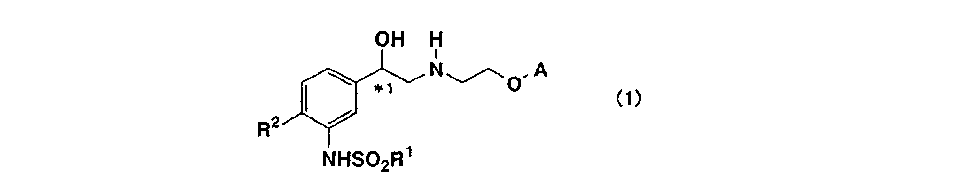 wherein R¹ represents a lower alkyl group or a benzyl group; R² represents a hydrogen atom, a halogen atom or a hydroxyl group; *1 represents an asymmetric carbon atom; and A represents one of the following groups: 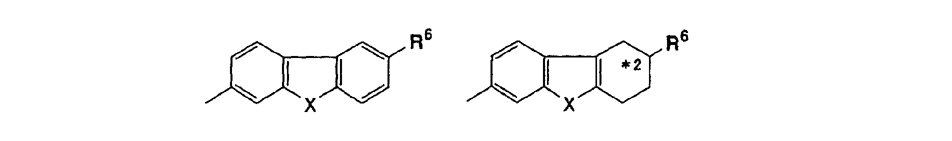 wherein X represents NH, O or S; R⁶ represents a hydrogen atom, a hydroxyl group, an amino group or an acetylamino group; and *2 represents an asymmetric carbon atom when R⁶ is not a hydrogen atom,
said process comprising:
chlorinating a compound of the formula (7): 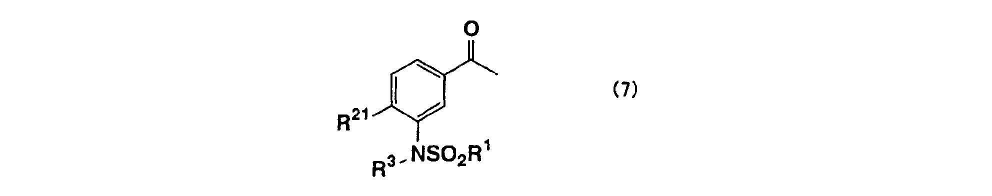 wherein R¹ is as defined above; R²¹ represents a hydrogen atom, a halogen atom or a protected hydroxyl group; and R³ represents an amino-protecting group, to give a compound of the formula (6): 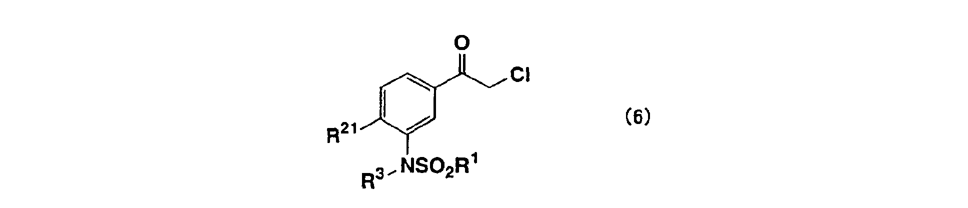 wherein R¹, R²¹ and R³ are as defined above; and then,
reducing the compound of the formula (6) to give a chlorohydrin compound of the formula (5): 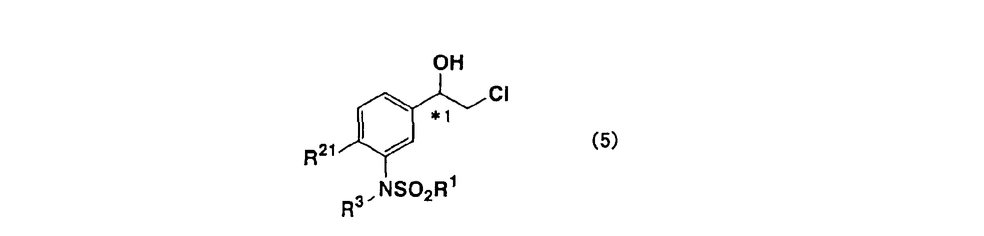 wherein R¹, R²¹, R³ and *1 are as defined above; and then,
treating the chlorohydrin compound of the formula (5) with an alkali to give an epoxy compound of the formula (4): 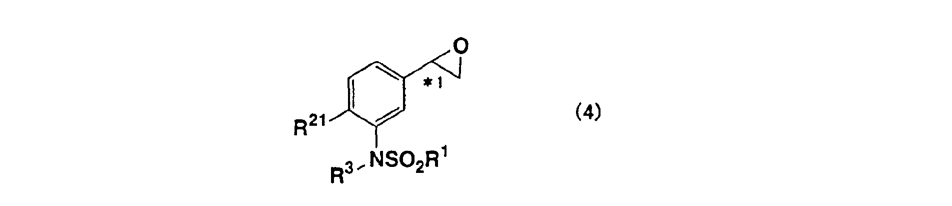 wherein R¹, R²¹, R³ and *1 are as defined above; and then,
reacting the epoxy compound of the formula (4) with a compound of the formula (8): 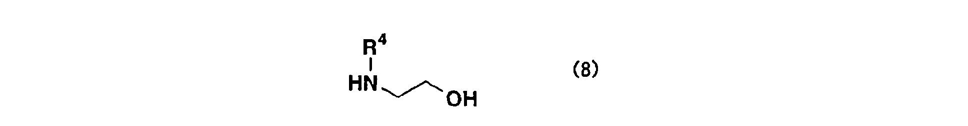 wherein R⁴ represents an amino-protecting group; to give a dialcohol compound of the formula (3): 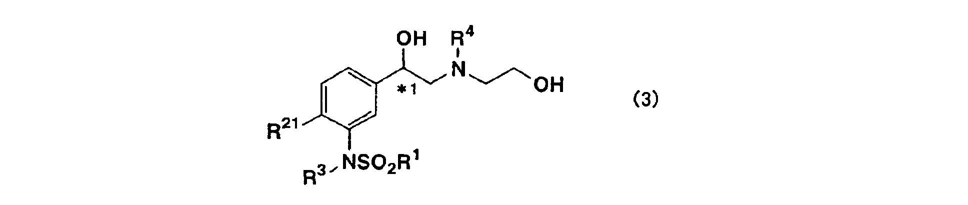 wherein R¹, R²¹, R³, R⁴ and *1 are as defined above;
brominating the primary alcohol, and then reacting the brominated product with a compound represented by A'-OH wherein A' represents one of the following groups: 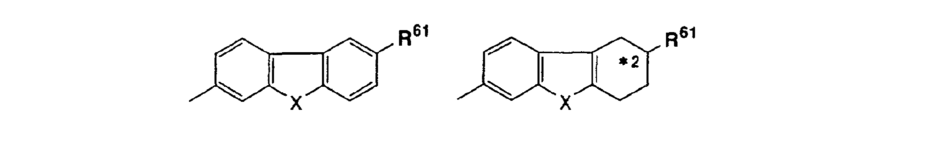 wherein X represents NH, O or S; R⁶¹ represents a hydrogen atom, a protected hydroxyl group, a protected amino group or an acetylamino group; and *2 represents an asymmetric carbon atom when R⁶¹ is not a hydrogen atom, to give an amino alcohol compound of the formula (2): 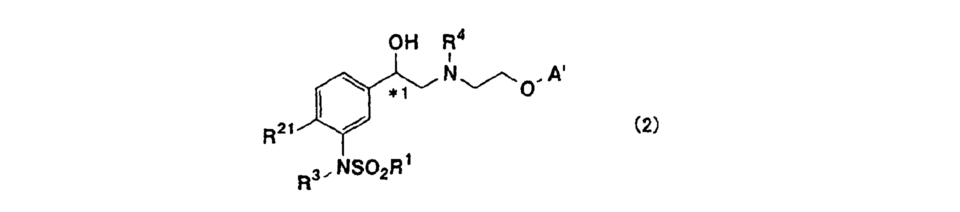 wherein R¹, R²¹, R³, R⁴, A' and *1 are as defined above; and then, simultaneously or sequentially removing the protecting groups of the compound of the formula (2) to give a compound of the formula (1).

3. The process as claimed in claim 1 or 2, wherein in the step of reducing the compound of the formula (6) to give a chlorohydrin compound, the compound of the formula (6) is asymmetrically reduced, and that the chlorohydrin compound of the formula (5) and the compound of the formula (1) are one of their optical isomers.

4. A process for the preparation of a compound of the formula (1): 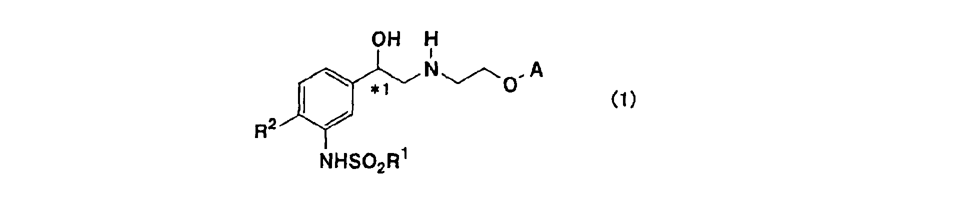 wherein R¹ represents a lower alkyl group or a benzyl group; R² represents a hydrogen atom, a halogen atom or a hydroxyl group; *1 represents an asymmetric carbon atom; and A represents one of the following groups: 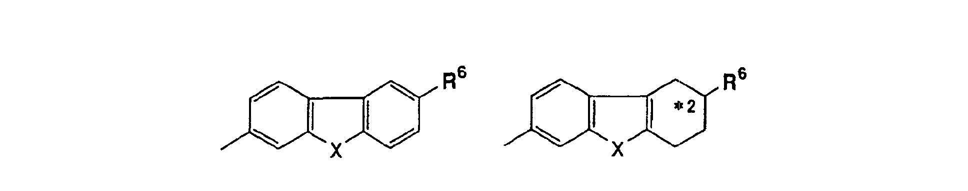 wherein X represents NH, O or S; R⁶ represents a hydrogen atom, a hydroxyl group, an amino group or an acetylamino group; and *2 represents an asymmetric carbon atom when R⁶ is not a hydrogen atom,
said process comprising:
reacting an epoxy compound of the formula (4): 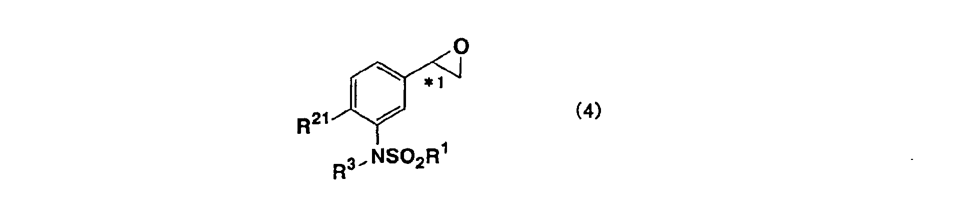 wherein R¹ and * 1 are as defined above; R²¹ represents a hydrogen atom, a halogen atom or a protected hydroxyl group; and R³ represents an amino-protecting group, with a compound of the formula (9): 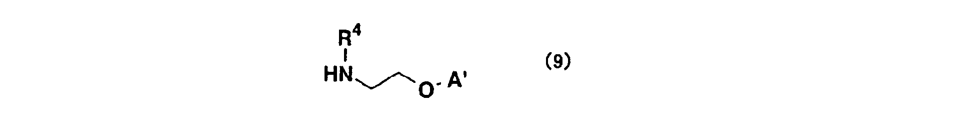 wherein R⁴ represents an amino-protecting group and A' represents one of the following groups: 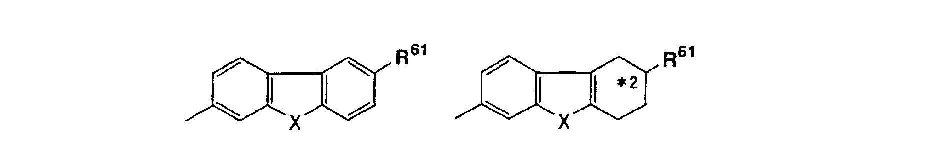 wherein X represents NH, O or S; R⁶¹ represents a hydrogen atom, a protected hydroxyl group, a protected amino group or an acetylamino group; and *2 represents an asymmetric carbon atom when R⁶¹ is not a hydrogen atom, to give a compound of the formula (2): 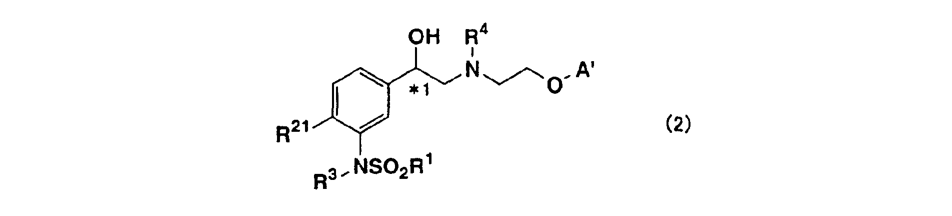 wherein R¹, R²¹, R³, R⁴, A' and *1 are as defined above; and then, simultaneously or sequentially removing the protecting groups of the compound of the formula (2) to give a compound of the formula (1).

5. A process for the preparation of a compound of the formula (5): 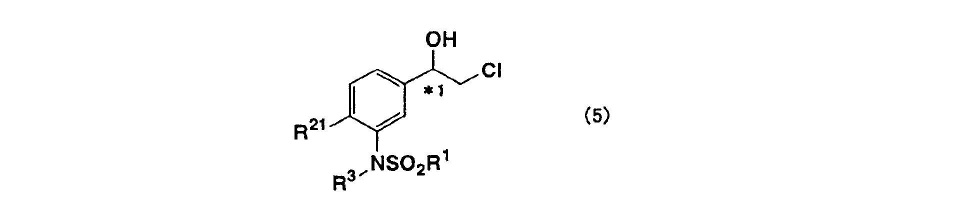 wherein R¹ represents a lower alkyl group or benzyl group; R²¹ represents a hydrogen atom, a halogen atom or a protected hydroxyl group; R³ represents an amino-protecting group and *1 represents an asymmetric carbon atom, which comprises reducing a compound of the formula (6): 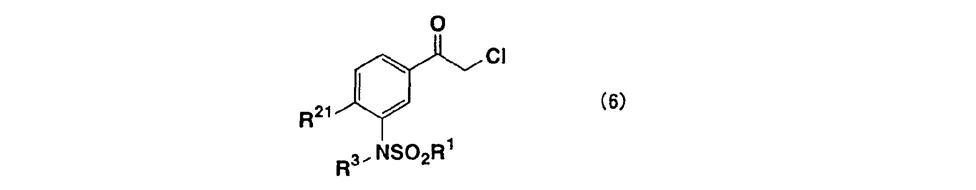 wherein R¹, R²¹ and R³ are as defined above, to give a compound of the formula (5).

6. The process as claimed in claim 5, wherein in the step of reducing the compound of the formula (6), the compound of the formula (6) is asymmetrically reduced, and the compound of the formula (5) is one of its optical isomers.

7. A compound of the formula (6): 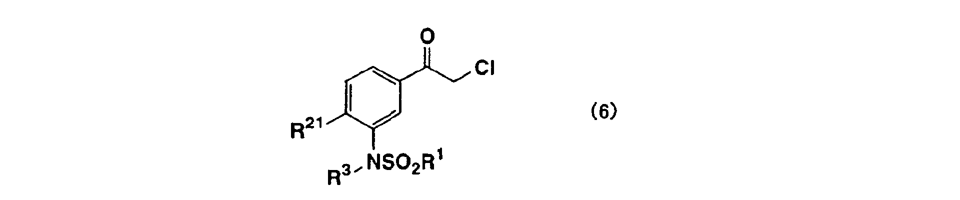 wherein R¹ represents a lower alkyl group or a benzyl group; R²¹ represents a hydrogen atom, a halogen atom or a protected hydroxyl group; and R³ represents an amino-protecting group .

8. A compound of the formula (5): 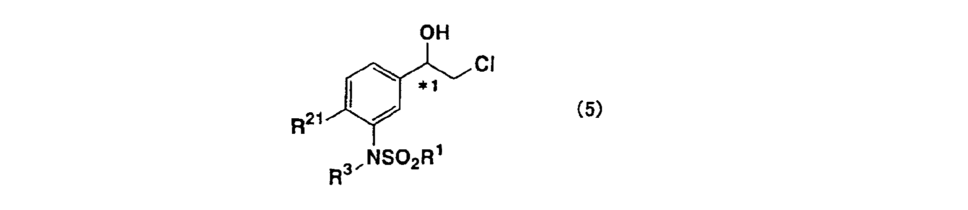 wherein R¹ represents a lower alkyl group or a benzyl group; R²¹ represents a hydrogen atom, a halogen atom or a protected hydroxyl group; R³ represents an amino-protecting group; and * 1 represents an asymmetric carbon atom.

9. A compound of the formula (4): 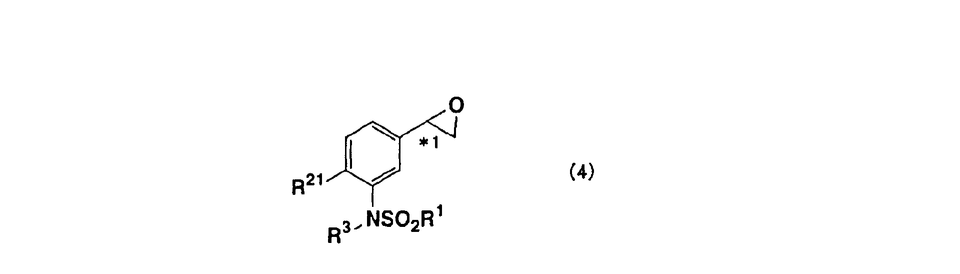 wherein R¹ represents a lower alkyl group or a benzyl group; R²¹ represents a hydrogen atom, a halogen atom or a protected hydroxyl group; R³ represents an amino-protecting group; and *1 represents an asymmetric carbon atom.

10. A compound of the formula (3) or a salt thereof: 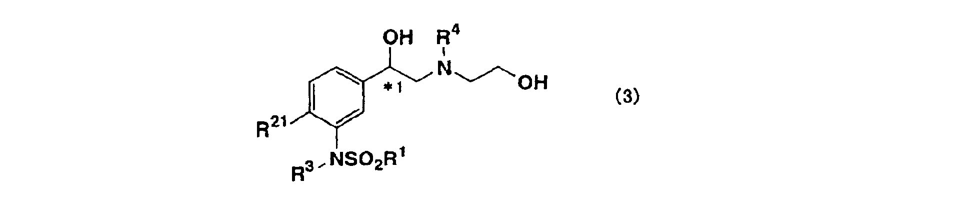 wherein R¹ represents a lower alkyl group or a benzyl group; R²¹ represents a hydrogen atom, a halogen atom or a protected hydroxyl group; R³ and R⁴ each represent an amino-protecting group; and *1 represents an asymmetric carbon atom.

11. A compound of the formula (10): 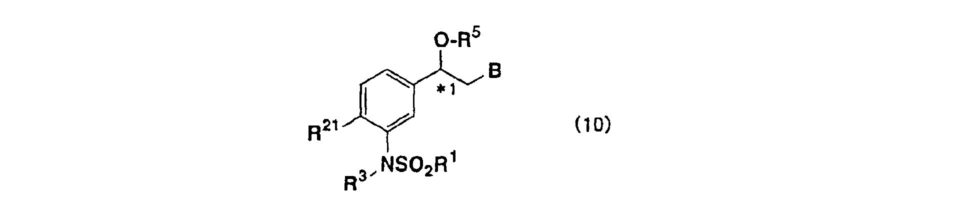 wherein R¹ represents a lower alkyl group or a benzyl group; R²¹ represents a hydrogen atom, a halogen atom or a protected hydroxyl group; R³ represents an amino-protecting group; R⁵ represents a hydroxyl-protecting group; B represents a halogen atom and *1 represents an asymmetric carbon atom.
